# EUROPEAN PATENT APPLICATION

(11) **EP 4 835 478 A1**
(43) Date of publication of application: **04.11.2026**
(21) Application number: 24912883.6
(22) Date of filing: 25.12.2024
(51) Int. Cl.: G01N 33/68, G01N 33/48

(54) **METHOD FOR EVALUATING INFLAMMATION AFTER PARTURITION**

(30) Priority: 28.12.2023 JP 2023222934
(71) Applicant: AJINOMOTO CO., INC., Chuo-ku Tokyo 104-8315 (JP)
(72) Inventor: NATSUME, Manami, Kawasaki-shi, Kanagawa 210-8681 (JP); NAKAGAWA, Kazuki, Kawasaki-shi, Kanagawa 210-8681 (JP); SUGIMOTO, Yusuke, Kawasaki-shi, Kanagawa 210-8681 (JP); IMAIZUMI, Akira, Kawasaki-shi, Kanagawa 210-8681 (JP)
(74) Representative: Mewburn Ellis LLP
(86) International application number: PCT/JP2024/045856
(87) International publication number: WO 2025/142997

(57) **Abstract**

Provided are an evaluating method and the like capable of providing reliable information on a state of postpartum inflammation before parturition. In the present embodiment, a state of inflammation in a ruminant after parturition is evaluated using at least one value of concentration values of 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) in blood of the ruminant before parturition, test values of 37 kinds of biochemistries (ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, kynurenine) in the blood, and measurement values of four kinds of measurement items (d-ROMs value, BAP value, BAP value/d-ROMs value, d-ROMs value/BAP value) obtained from the blood.

## Description

### TECHNICAL FIELD

The present invention relates to an evaluating method of postpartum inflammation, a calculating method, an evaluating apparatus, a calculating apparatus, an evaluating program, a calculating program, a recording medium, an evaluating system, and a terminal apparatus.

### BACKGROUND ART

The period called the perinatal period of about three weeks before and after parturition is a critical period for a cow. In the period, metabolic diseases, for example, ketosis, milk fever, placental retention, abomasum displacement, metritis, hoof disease, and mastitis, are often developed. Recently, it has been reported that inflammatory conditions may be responsible for many of these diseases (Nonpatent Literature 1).

Patent Document 1 describes an evaluating method and the like of postpartum ketosis.

Mastitis is diagnosed after parturition by the appearance of the udder during the lactation period, changes in milk properties, bacterial tests, and somatic cell counts (SCC).

Items showing differences in concentration in blood and urine collected before parturition between cows normal after parturition and cows developing mastitis have been reported (Nonpatent Literatures 2 to 5).

Metritis and endometritis are diagnosed after parturition by examination of the mucosa, cytology, and the like and clinical findings.

Items showing differences in concentration in blood collected before parturition between cows normal after parturition and cows developing metritis have been reported (Nonpatent Literatures 6 to 11).

It has been reported that d-ROMs as an index of oxidative stress and BAP as an index of antioxidant capacity fluctuate 52 weeks prior to the onset of inflammatory conditions in humans (Nonpatent Literatures 12 to 13 and 19). On the other hand, it has been reported that these values fluctuate in cow ketosis at 14 days before parturition and 15 days after parturition (Nonpatent Literature 20). These findings suggest that d-ROMs, BAP, and the ratio between d-ROMs and BAP may be effective in predicting diseases before parturition and in understanding inflammatory conditions after parturition.

Blood haptoglobin is known as an index of inflammatory conditions. In dairy cows with high blood concentrations of haptoglobin and considered to be in inflammatory conditions, lower milk yield and lower conception rates have been observed (Nonpatent Literatures 1 and 14 to 17). Although several different criteria have been presented depending on the disease as criterion values for haptoglobin, uniform knowledge has not yet been disseminated.

IL-1β and serum albumin A are known as indices of inflammatory conditions (Nonpatent Literature 5). Although criteria have been presented depending on the disease as criterion values, uniform knowledge has not yet been disseminated.

LBP is known as an index of inflammatory conditions (Nonpatent Literature 18). Although criteria have been presented depending on the disease as criterion values, uniform knowledge has not yet been disseminated.

### PRIOR ART REFERENCES

### PATENT DOCUMENTS

Patent Document 1: WO 2018/003638

### NONPATENT LITERATURES

Nonpatent Literature 1: Horst, E. A., et al. (2021). "Invited review: The influence of immune activation on transition cow health and performance-A critical evaluation of traditional dogmas." J Dairy Sci 104(8): 8380-8410.
Nonpatent Literature 2: Hu, H., et al. (2021). "Application of Metabolomics in Diagnosis of Cow Mastitis: A Review." Front Vet Sci 8: 747519.
Nonpatent Literature 3: Zhang, G., et al. (2022). "Identification of Serum-Predictive Biomarkers for Subclinical Mastitis in Dairy Cows and New Insights into the Pathobiology of the Disease." J Agric Food Chem 70(5): 1724-1746.
Nonpatent Literature 4: Sakemi, Y., et al. (2011). "Interleukin-6 in quarter milk as a further prediction marker for bovine subclinical mastitis." J Dairy Res 78(1): 118-121.
Nonpatent Literature 5: Dervishi, E., et al. (2015). "Innate immunity and carbohydrate metabolism alterations precede occurrence of subclinical mastitis in transition dairy cows." J Anim Sci Technol 57: 46.
Nonpatent Literature 6: Dervishi, E., et al. (2016). "Alterations in innate immunity reactants and carbohydrate and lipid metabolism precede occurrence of metritis in transition dairy cows." Res Vet Sci 104: 30-39.
Nonpatent Literature 7: Dervishi, E., et al. (2018). "Urine metabolic fingerprinting can be used to predict the risk of metritis and highlight the pathobiology of the disease in dairy cows." Metabolomics 14(6): 83.
Nonpatent Literature 8: Wisnieski, L., et al. (2019). "Predictive models for early lactation diseases in transition dairy cattle at dry-off." Prev Vet Med 163: 68-78.
Nonpatent Literature 9: Paiano, R. B., et al. (2021). "Metritis in dairy cows is preceded by alterations in biochemical profile prepartum and at parturition." Res Vet Sci 135: 167-174.
Nonpatent Literature 10: Chebel, R. C. (2021). "Predicting the risk of retained fetal membranes and metritis in dairy cows according to prepartum hemogram and immune and metabolic status." Prev Vet Med 187: 105204.
Nonpatent Literature 11: Casaro, S., et al. (2023). "Blood metabolomics and impacted cellular mechanisms during transition into lactation in dairy cows that develop metritis." J Dairy Sci 106(11): 8098-8109.
Nonpatent Literature 12: Nakajima, A., et al. (2019). "Serum levels of reactive oxygen metabolites at 12 weeks during tocilizumab therapy are predictive of 52 weeks-disease activity score-remission in patients with rheumatoid arthritis." BMC Rheumatol 3: 48.
Nonpatent Literature 13: Masaki, N., et al. (2016). "Usefulness of the d-ROMs test for prediction of cardiovascular events." Int J Cardiol 222: 226-232.
Nonpatent Literature 14: Dubuc, J., et al. (2010). "Risk factors for postpartum uterine diseases in dairy cows." J Dairy Sci 93(12): 5764-5771.
Nonpatent Literature 15: Huzzey, J. M., et al. (2009). "Short communication: Haptoglobin as an early indicator of metritis." J Dairy Sci 92(2): 621-625.
Nonpatent Literature 16: Kerwin, A. L., et al. (2022). "Transition cow nutrition and management strategies of dairy herds in the northeastern United States: Part II-Associations of metabolic- and inflammation-related analytes with health, milk yield, and reproduction." J Dairy Sci 105(6): 5349-5369.
Nonpatent Literature 17: Kerwin, A. L., et al. (2022). "Transition cow nutrition and management strategies of dairy herds in the northeastern United States: Part I-Herd description and performance characteristics." J Dairy Sci 105(6): 5327-5348.
Nonpatent Literature 18: Abuajamieh, M., et al. (2016). "Inflammatory biomarkers are associated with ketosis in periparturient Holstein cows." Res Vet Sci 109: 81-85.
Nonpatent Literature 19: Nakajima, A., et al. (2019). "Serum levels of reactive oxygen metabolites at 12 weeks during tocilizumab therapy are predictive of 52 weeks-disease activity score-remission in patients with rheumatoid arthritis." BMC Rheumatol 3: 48.
Nonpatent Literature 20: Kenichi Shibano, et al. (2021) "Oxidative Stress Markers and Biochemical Characteristics of Cows with Ketosis", Journal of the Japan Veterinary Medical Association 74, 59-63.

### DISCLOSURE OF INVENTION

### PROBLEM TO BE SOLVED BY THE INVENTION

If it is possible that the risk of developing inflammation after parturition be diagnosed in advance before parturition, for example, prophylactic nutritional intervention before parturition is considered to be able to reduce the development of inflammation and thereby contribute to efficient production for dairy farmers.

As described above, it has been reported that in cows that develop inflammation after parturition, there are fluctuations in some components even before parturition. As for the prepartum diagnosis of the risk of developing inflammation after parturition, however, the problem is that there is no technique actually used in farms for diagnosing the risk.

In view of the above, it is an object of the present invention to provide an evaluating method, a calculating method, an evaluating apparatus, a calculating apparatus, an evaluating program, a calculating program, a recording medium, an evaluating system, and a terminal apparatus capable of providing reliable information on a state of postpartum inflammation before parturition.

### MEANS FOR SOLVING PROBLEM

To solve the problem and achieve the object, an evaluating method according to one aspect of the present invention includes an evaluating step of evaluating a state of inflammation in a ruminant after parturition, using at least one value of concentration values of 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) in blood of the ruminant before parturition, test values of 37 kinds of biochemistries (that is, biochemical parameters, specifically, ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, kynurenine) in the blood, and measurement values of four kinds of measurement items (d-ROMs value (oxidative stress value), BAP value (antioxidant capacity value), BAP value/d-ROMs value, d-ROMs value/BAP value) obtained from the blood, or using a value of a formula calculated using the at least one value and the formula including an explanatory variable to be substituted with the at least one value.

In the present description, various amino acids, various blood boichemistries, and various measurement items are mainly written in abbreviations, and the formal names of these are as follows.

### (Abbreviation for amino acid) (Formal name)

Ala Alanine
Arg Arginine
Asn Asparagine
Asp Aspartic acid
BCAA Branched chain amino acids
Cit Citrulline
Cys Cystine
Gln Glutamine
Glu Glutamic acid
Gly Glycine
His Histidine
Ile Isoleucine
Leu Leucine
Lys Lysine
Met Methionine
3MeHis 3-Methyl histidine
Orn Ornithine
Phe Phenylalanine
Pro Proline
Ser Serine
Tau Taurine
Thr Threonine
Trp Tryptophan
Tyr Tyrosine
Val Valine

### (Abbreviation for blood biochemistry) (Formal name)

ALB Albumin
ALT Alanine transaminase
AST Aspartate Aminotransferase
BHBA β-Hydroxybutyric acid
BUN Blood urea nitrogen
Ca Calcium
gGTP γ-glutamyltransferase
Glc Glucose
Glob Globulin
NEFA Non esterified fatty acid
T-Bil Total bilirubin
TCHO Total cholesterol
TG Triglyceride
TP Total protein
P Phosphorus
Na Sodium
K Potassium
Cl Chlorine
GGT γ-Glutamyltransferase
ALP Alkaline phosphatase
LDH Lactate dehydrogenase
CPK Creatine phosphokinase
LDL-C LDL cholesterol
HDL-C HDL cholesterol
Mg Magnesium
AMY Amylase
PA Pyruvic acid
LA Lactic acid
CRE Creatine
LBP Lipopolysaccharide Binding Protein
Il-1β Interleukin-1β
IL-6 Interleukin-6
TNF-α Tumor Necrosis Factor-α

### (Abbreviation for measurement item) (Formal name)

d-ROMs Diacron-Reactive oxygen metabolites
BAP Biological antioxidant potential

The inflammation may be metritis, mastitis, hypocalcemia, milk fever, abomasum displacement, or lameness.

The ruminant may be a dairy cow or a beef cow.

The evaluating step may be executed in a control unit of an information processing apparatus.

A calculating method according to one aspect of the present invention includes a calculating step of calculating a value of a formula for evaluating a state of inflammation in a ruminant after parturition, using (i) at least one value of concentration values of the 25 kinds of amino acids in blood of the ruminant before parturition, test values of the 37 kinds of biochemistries in the blood, and measurement values of the four kinds of measurement items obtained from the blood, and (ii) the formula including an explanatory variable to be substituted with the at least one value.

The calculating step may be executed in a control unit of an information processing apparatus.

An evaluating apparatus according to one aspect of the present invention includes an evaluating unit that evaluates a state of inflammation in a ruminant after parturition, using at least one value of concentration values of the 25 kinds of amino acids in blood of the ruminant before parturition, test values of the 37 kinds of biochemistries in the blood, and measurement values of the four kinds of measurement items obtained from the blood, or using a value of a formula calculated using the at least one value and the formula including an explanatory variable to be substituted with the at least one value.

The evaluating apparatus according to another aspect of the present invention may be communicably connected via a network to a terminal apparatus that provides the at least one value or the value of the formula. The evaluating apparatus may further include a data receiving unit that receives the at least one value or the value of the formula transmitted from the terminal apparatus, and a result sending unit that transmits an evaluation result obtained by the evaluating unit to the terminal apparatus. The evaluating unit may use the at least one value or the value of the formula received by the data receiving unit.

A calculating apparatus according to one aspect of the present invention includes a calculating unit that calculates a value of a formula for evaluating a state of inflammation in a ruminant after parturition, using (i) at least one value of concentration values of the 25 kinds of amino acids in blood of the ruminant before parturition, test values of the 37 kinds of biochemistries in the blood, and measurement values of the four kinds of measurement items obtained from the blood, and (ii) the formula including an explanatory variable to be substituted with the at least one value.

An evaluating program according to one aspect of the present invention includes programmed instructions for causing an information processing apparatus to execute an evaluating method including an evaluating step of evaluating a state of inflammation in a ruminant after parturition, using at least one value of concentration values of the 25 kinds of amino acids in blood of the ruminant before parturition, test values of the 37 kinds of biochemistries in the blood, and measurement values of the four kinds of measurement items obtained from the blood, or using a value of a formula calculated using the at least one value and the formula including an explanatory variable to be substituted with the at least one value.

A calculating program according to one aspect of the present invention includes programmed instructions for causing an information processing apparatus to execute a calculating method including a calculating step of calculating a value of a formula for evaluating a state of inflammation in a ruminant after parturition, using (i) at least one value of concentration values of the 25 kinds of amino acids in blood of the ruminant before parturition, test values of the 37 kinds of biochemistries in the blood, and measurement values of the four kinds of measurement items obtained from the blood, and (ii) the formula including an explanatory variable to be substituted with the at least one value.

A recording medium according to one aspect of the present invention is a computer-readable recording medium that stores the evaluating program or the calculating program. In other words, the recording medium according to one aspect of the present invention is a non-transitory tangible computer-readable recording medium including the programmed instructions for causing an information processing apparatus to execute the evaluating method or the calculating method.

An evaluating system according to one aspect of the present invention is an evaluating system including an evaluating apparatus and a terminal apparatus that are connected to each other communicatively via a network. The terminal apparatus includes (I) a data sending unit that transmits, to the evaluating apparatus, (i) at least one value of concentration values of the 25 kinds of amino acids in blood of a ruminant before parturition, test values of the 37 kinds of biochemistries in the blood, and measurement values of the four kinds of measurement items obtained from the blood or (ii) a value of a formula calculated using the at least one value and the formula including an explanatory variable to be substituted with the at least one value, and (II) a result receiving unit that receives an evaluation result on a state of inflammation after parturition transmitted from the evaluating apparatus. The evaluating apparatus includes (I) a data receiving unit that receives the at least one value or the value of the formula transmitted from the terminal apparatus, (II) an evaluating unit that evaluates the state of the inflammation in the ruminant after parturition, using the at least one value or the value of the formula received by the data receiving unit, and (III) a result sending unit that transmits the evaluation result obtained by the evaluating unit to the terminal apparatus.

A terminal apparatus according to one aspect of the present invention includes a result obtaining unit that obtains an evaluation result on a state of inflammation after parturition. The evaluation result is a result of evaluating the state of the inflammation in a ruminant after parturition, using at least one value of concentration values of the 25 kinds of amino acids in blood of the ruminant before parturition, test values of the 37 kinds of biochemistries in the blood, and measurement values of the four kinds of measurement items obtained from the blood, or using a value of a formula calculated using the at least one value and the formula including an explanatory variable to be substituted with the at least one value.

The terminal apparatus according to another aspect of the present invention may be communicatively connected via a network to an evaluating apparatus that evaluates a state of inflammation after parturition. The terminal apparatus may further include a data sending unit that transmits the at least one value or the value of the formula to the evaluating apparatus. The result obtaining unit may receive the evaluation result transmitted from the evaluating apparatus.

The evaluating method according to another aspect of the present invention may further include a proposing step of proposing a prophylactic measure for a cow evaluated at the evaluating step as having a high possibility of suffering from the metabolic disease after parturition.

The prophylactic measure may be at least one selected from the group consisting of administration of Rumen Protect amino acid, administration of a feed additive, administration of a drug, and veterinary diagnosis.

The feed additive may be at least one selected from the group consisting of PH adjusters, ion balance adjusters, mycotoxin adsorbents, propionate analogs such as calcium propionate, vitamins, minerals, amino acids, fatty acids, urea, probiotics, yeasts, enzymes, antibiotics, antioxidants, antibacterial agents, and organic acids.

In the proposing step, statistical causal inference may be performed to infer a cause of disease for a cow evaluated as having a possibility of suffering from the metabolic disease, and a prophylactic measure corresponding to the cause may be proposed.

### EFFECT OF THE INVENTION

According to the present invention, the state of inflammation in a ruminant after parturition is evaluated using at least one value of the concentration values of the 25 kinds of amino acids in blood of the ruminant before parturition, the test values of the 37 kinds of biochemistries in the blood, and the measurement values of the four kinds of measurement items obtained from the blood, whereby reliable information on the state of postpartum inflammation can be provided before parturition. The dairy farmers can achieve the reduction of development of postpartum inflammation by prophylactic nutritional intervention before parturition based on the information provided by the present invention. In other words, the present invention can contribute to efficient production for the dairy farmers.

### BRIEF DESCRIPTION OF DRAWINGS

FIG. 1 is a principle configurational diagram showing a basic principle of a first embodiment;
FIG. 2 is a principle configurational diagram showing a basic principle of a second embodiment;
FIG. 3 is a diagram showing an example of an entire configuration of a present system;
FIG. 4 is a diagram showing another example of an entire configuration of the present system;
FIG. 5 is a block diagram showing an example of a configuration of an evaluating apparatus 100 in the present system;
FIG. 6 is a chart showing an example of information stored in a blood data file 106a;
FIG. 7 is a chart showing an example of information stored in an index state information file 106b;
FIG. 8 is a chart showing an example of information stored in a designated index state information file 106c;
FIG. 9 is a chart showing an example of information stored in a formula file 106d1;
FIG. 10 is a chart showing an example of information stored in an evaluation result file 106e;
FIG. 11 is a block diagram showing a configuration of an evaluating part 102d;
FIG. 12 is a block diagram showing an example of a configuration of a client apparatus 200 in the present system;
FIG. 13 is a block diagram showing an example of a configuration of a database apparatus 400 in the present system;
FIG. 14 is a table of linear regression models extracted in Example 4;
FIG. 15 is a table of linear regression models extracted in Example 4;
FIG. 16 is a table of linear regression models extracted in Example 4;
FIG. 17 is a table of linear regression models extracted in Example 4;
FIG. 18 is a table of logistic regression models extracted in Example 4;
FIG. 19 is a table of logistic regression models extracted in Example 4;
FIG. 20 is a table of logistic regression models extracted in Example 4;
FIG. 21 is a table of logistic regression models extracted in Example 4;
FIG. 22 is a table of logistic regression models extracted in Example 4;
FIG. 23 is a table of logistic regression models extracted in Example 4;
FIG. 24 is a table of logistic regression models extracted in Example 4;
FIG. 25 is a table of logistic regression models extracted in Example 4;
FIG. 26 is a table of logistic regression models extracted in Example 4;
FIG. 27 is a table of logistic regression models extracted in Example 4;
FIG. 28 is a table of logistic regression models extracted in Example 5;
FIG. 29 is a table of logistic regression models extracted in Example 5;
FIG. 30 is a table of logistic regression models extracted in Example 5;
FIG. 31 is a table of logistic regression models extracted in Example 5;
FIG. 32 is a table of logistic regression models extracted in Example 5;
FIG. 33 is a table of logistic regression models extracted in Example 5;
FIG. 34 is a table of logistic regression models extracted in Example 5;
FIG. 35 is a table of logistic regression models for milk fever discrimination extracted in Example 6;
FIG. 36 is a table of logistic regression models for milk fever discrimination and logistic regression models for abomasum displacement extracted in Example 6;
FIG. 37 is a table of logistic regression models for abomasum displacement extracted in Example 6;
FIG. 38 is a table of logistic regression models for abomasum displacement extracted in Example 6;
FIG. 39 is a table of logistic regression models for metritis discrimination extracted in Example 6;
FIG. 40 is a table of logistic regression models for metritis discrimination extracted in Example 6;
FIG. 41 is a table of logistic regression models for metritis discrimination extracted in Example 6;
FIG. 42 is a table of logistic regression models for metritis discrimination extracted in Example 6;
FIG. 43 is a table of logistic regression models for metritis discrimination extracted in Example 6;
FIG. 44 is a table of logistic regression models for mastitis discrimination extracted in Example 6;
FIG. 45 is a table of logistic regression models for mastitis discrimination extracted in Example 6;
FIG. 46 is a table of logistic regression models for mastitis discrimination extracted in Example 6;
FIG. 47 is a table of logistic regression models for mastitis discrimination extracted in Example 6;
FIG. 48 is a table of logistic regression models for mastitis discrimination extracted in Example 6;
FIG. 49 is a table of logistic regression models for lameness discrimination extracted in Example 6;
FIG. 50 is a table of logistic regression models for lameness discrimination and logistic regression models for inflammatory disease discrimination extracted in Example 6;
FIG. 51 is a table of logistic regression models for inflammatory disease discrimination extracted in Example 6;
FIG. 52 is a table of logistic regression models for inflammatory disease discrimination extracted in Example 6;
FIG. 53 is a table of logistic regression models for inflammatory disease discrimination extracted in Example 6;
FIG. 54 is a table of logistic regression models for inflammatory disease discrimination extracted in Example 6;
FIG. 55 is a table of logistic regression models for inflammatory disease discrimination extracted in Example 6;
FIG. 56 is a table of logistic regression models for inflammatory disease discrimination extracted in Example 6;
FIG. 57 is a table of logistic regression models for inflammatory disease discrimination extracted in Example 6;
FIG. 58 is a table of logistic regression models for inflammatory disease discrimination extracted in Example 6;
FIG. 59 is a table of logistic regression models for inflammatory disease discrimination extracted in Example 6 and linear regression models to predict serum amyloid A concentration 7 days after the date of parturition extracted in Example 7;
FIG. 60 is a table of linear regression models to predict serum amyloid A concentration 7 days after the date of parturition extracted in Example 7;
FIG. 61 is a table of linear regression models to predict serum amyloid A concentration 7 days after the date of parturition extracted in Example 7;
FIG. 62 is a table of linear regression models to predict serum amyloid A concentration 7 days after the date of parturition extracted in Example 7;
FIG. 63 is a table of linear regression models to predict serum amyloid A concentration 7 days after the date of parturition extracted in Example 7;
FIG. 64 is a table of linear regression models to predict IL-1β concentration 7 days after the date of parturition extracted in Example 7;
FIG. 65 is a table of linear regression models to predict IL-1β concentration 7 days after the date of parturition extracted in Example 7;
FIG. 66 is a table of linear regression models to predict IL-1β concentration 7 days after the date of parturition extracted in Example 7;
FIG. 67 is a table of linear regression models to predict IL-1β concentration 7 days after the date of parturition and linear regression models to predict d-ROMs value 7 days after the date of parturition extracted in Example 7;
FIG. 68 is a table of linear regression models to predict d-ROMs value 7 days after the date of parturition extracted in Example 7;
FIG. 69 is a table of linear regression models to predict d-ROMs value 7 days after the date of parturition extracted in Example 7;
FIG. 70 is a table of linear regression models to predict d-ROMs value 7 days after the date of parturition extracted in Example 7;
FIG. 71 is a table of linear regression models to predict BAP value 7 days after the date of parturition extracted in Example 7;
FIG. 72 is a table of linear regression models to predict BAP value 7 days after the date of parturition extracted in Example 7;
FIG. 73 is a table of linear regression models to predict BAP value 7 days after the date of parturition extracted in Example 7;
FIG. 74 is a table of linear regression models to predict BAP value 7 days after the date of parturition and linear regression models to predict d-ROMs value/BAP value 7 days after the date of parturition extracted in Example 7; and
FIG. 75 is a table of linear regression models to predict d-ROMs value/BAP value 7 days after the date of parturition extracted in Example 7.

### BEST MODE(S) FOR CARRYING OUT THE INVENTION

Hereinafter, an embodiment (first embodiment) of the evaluating method and the calculating method according to the present invention and an embodiment (second embodiment) of the evaluating apparatus, the calculating apparatus, the evaluating method, the calculating method, the evaluating program, the calculating program, the recording medium, the evaluating system, and the terminal apparatus according to the present invention are described in detail with reference to the drawings. The present invention is not limited to these embodiments.

### First Embodiment

### 1-1. Outline of first embodiment

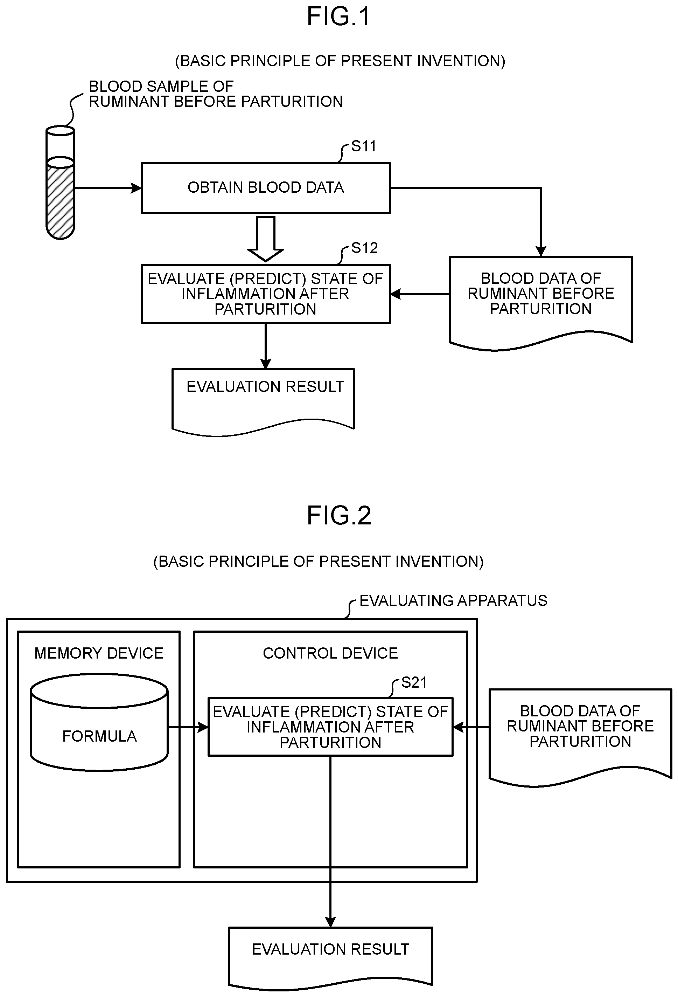

Here, an outline of the first embodiment will be described with reference to FIG. 1. FIG. 1 is a principle configurational diagram showing a basic principle of the first embodiment.

First, blood data including at least one value of concentration values of the 25 kinds of amino acids in blood (including, for example, plasma or serum) extracted from a ruminant as a subject to be evaluated (for example, dairy cow) before parturition (for example, a certain period before the expected date of parturition) and test values of the 37 kinds of biochemistries in the blood, and measurement values of the four kinds of measurement items obtained from the blood (any one or two or more values selected from the concentration values of the 25 kinds of amino acids, the test values of the 37 kinds of biochemistries, and the measurement values of the four kinds of measurement items) is obtained (step S11).

At step S11, for example, the blood data measured by a company or other organization that measures the concentration values or the test values may be obtained. In addition, for example, the following measuring method of (A), (B), or (C) may be used to measure the concentration values or the test values from the blood extracted from the subject to be evaluated before parturition to obtain the blood data. Here, the unit of concentration value may be molar concentration, weight concentration, enzyme activity, or one obtained by addition, subtraction, multiplication, and division of any constant with these concentrations.
(A) Plasma is separated from blood by centrifuging the collected blood sample. All plasma samples are frozen and stored at -80°C until the concentration value is measured. At the time of measuring the concentration value, 0.02N hydrochloric acid is added to perform a protein removal treatment by ultrafiltration, pre-column derivatization is then performed using a labeled reagent (3-aminopyridyl-N-hydroxysuccinimidyl carbamate), and the concentration value is analyzed by liquid chromatograph mass spectrometer (LC/MS) (see International Publication WO 2003/069328 and International Publication WO 2005/116629).
(B) Plasma is separated from blood by centrifuging the collected blood sample. All plasma samples are frozen and stored at -80°C until the concentration value is measured. At the time of measuring the concentration value, 0.02N hydrochloric acid is added to perform a protein removal treatment by ultrafiltration, and the concentration value is analyzed by an amino acid analyzer based on post-column derivatization using a ninhydrin reagent.
(C) Blood cell separation is performed on the collected blood sample by using a membrane, MEMS (Micro Electro Mechanical Systems) technology, or the principle of centrifugation, whereby plasma or serum is separated from the blood. A plasma or serum sample the concentration value of which is not measured immediately after obtaining the plasma or the serum is frozen and stored at -80°C until the concentration value is measured. At the time of measuring the concentration value, a molecule that reacts with or binds to a target amino acid or biochemistry, such as an enzyme or an aptamer, and the like are used to perform quantitative analysis and the like on an increasing or decreasing substance or a spectroscopic value by substrate recognition, whereby the concentration value is analyzed.

The state of inflammation (for example, metritis, mastitis, hypocalcemia, milk fever, abomasum displacement, or lameness) of the subject to be evaluated after parturition (for example, a certain period after the date of parturition) is evaluated (predicted/estimated), using the at least one value of the concentration values of the 25 kinds of amino acids, the test values of the 37 kinds of biochemistries, and the measurement values of the four kinds of measurement items included in the blood data obtained at step S11 (step S12). Before step S12 is executed, data such as missing values and outliers may be removed from the blood data obtained at step S11.

In this way, reliable information on the state of postpartum inflammation can be provided before parturition.

At step S12, the state of postpartum inflammation of the subject to be evaluated may be evaluated by calculating a value of a formula using (i) the at least one value of the concentration values of the 25 kinds of amino acids, the test values of the 37 kinds of biochemistries, and the measurement values of the four kinds of measurement items, and (ii) the formula including an explanatory variable to be substituted with the at least one value. The explanatory variable to be substituted with the concentration value, the test value, or the measurement value may be substituted with a value obtained by converting the concentration value, the test value, or the measurement value by the later-described method.

When the state of postpartum inflammation is evaluated, for example, a value related to the factors listed below having an impact on the development of inflammation may further be used in addition to the at least one value of the concentration values of the 25 kinds of amino acids, the test values of the 37 kinds of biochemistries, and the measurement values of the four kinds of measurement items. For example, an explanatory variable to be substituted with a value related to the factors listed below having an impact on the development of inflammation may further be included in the formula in addition to the explanatory variable to be substituted with the at least one value of the concentration values of the 25 kinds of amino acids, the test values of the 37 kinds of biochemistries, and the measurement values of the four kinds of measurement items.
- parity term for indicating parous cow or nulliparous cow (binary variable); and
- body weight, food intake, body condition score (BCS), air temperature, humidity, breeding density, and season

The concentration value or the value of the formula may be converted, for example, by the methods listed below, and the state of postpartum inflammation of the subject to be evaluated may be evaluated using the converted value.

The concentration value or the value of the formula may be converted such that a possible range of the concentration value or the value of the formula falls within a predetermined range (for example, the range from 0.0 to 1.0, the range from 0.0 to 10.0, the range from 0.0 to 100.0, or the range from -10.0 to 10.0), for example, by addition, subtraction, multiplication, and division of any given value with the concentration value or the value of the formula, by conversion of the concentration value or the value of the formula by a predetermined conversion method (for example, exponential transformation, logarithm transformation, angular transformation, square root transformation, probit transformation, reciprocal transformation, Box-Cox transformation, or power transformation), or by performing a combination of these computations on the concentration value or the value of the formula. For example, a value of an exponential function with the concentration value or the value of the formula as an exponent and Napier constant as the base may be further calculated (specifically, a value of p/(1-p) where a natural logarithm ln(p/(1-p)) is equal to the concentration value or the value of the formula when the probability p that the state of postpartum inflammation has a predetermined state (for example, the state where the blood concentration of haptoglobin exceeds the criterion value) is defined), and the value (specifically, the value of the probability p) may be further calculated by dividing the calculated value of the exponential function by the sum of 1 and the value of the exponential function.

The concentration value or the value of the formula may be converted such that the converted value is a particular value when a particular condition is met. For example, the concentration value or the value of the formula may be converted such that the converted value is 5.0 when the sensitivity is 95% and the converted value is 8.0 when the sensitivity is 80%.

As for the concentration value, after the concentration value is normally distributed for each amino acid, the concentration value may be standardized with a mean of 50 and a standard deviation of 10. As for the value of the formula, the value of the formula may be standardized with a mean of 50 and a standard deviation of 10.

These conversions may be applied to the test values and the measurement values.

Positional information about a position of a predetermined mark on a predetermined scale visually presented on a display device such as a monitor or a physical medium such as paper may be generated using the at least one value of the concentration values of the 25 kinds of amino acids, the test values of the 37 kinds of biochemistries, and the measurement values of the four kinds of measurement items (if the value is converted, the converted value) or the value of the formula (if the value of the formula is converted, the converted value), and the generated positional information may be treated as an evaluation result on the state of postpartum inflammation of the subject to be evaluated. The predetermined scale is for evaluating the state of postpartum inflammation and is, for example, a graduated scale at least marked with graduations corresponding to the upper limit value and the lower limit value in "a possible range of the concentration value, the test value, the measurement value or the value of the formula, or the converted value" or "part of the range". The predetermined mark corresponds to the concentration value, the test value, the measurement value or the value of the formula, or the converted value, and is, for example, a circle sign or a star sign.

If the at least one value of the concentration values of the 25 kinds of amino acids, the test values of the 37 kinds of biochemistries, and the measurement values of the four kinds of measurement items, or the value of the formula is lower than a predetermined value (for example, mean ±1SD, 2SD, 3SD, N quantile, N percentile, or a cutoff value the clinical significance of which is recognized) or is equal to or lower than the predetermined value or is equal to or higher than the predetermined value or is higher than the predetermined value, the subject to be evaluated may be evaluated as developing inflammation after parturition. In this case, instead of the concentration value or the value of the formula itself, a standard score may be used. For example, if the standard score is lower than the mean -2SD (when the standard score<30) or if the standard score is higher than the mean +2SD (when the standard score>70), the subject to be evaluated may be evaluated as developing inflammation after parturition.

The risk (possibility) that the subject to be evaluated develops the inflammation after parturition may be qualitatively evaluated.

Specifically, the subject may be classified into any one of a plurality of categories defined at least considering the risk of developing the inflammation after parturition, using (i) the at least one value of the concentration values of the 25 kinds of amino acids, the test values of the 37 kinds of biochemistries, and the measurement values of the four kinds of measurement items and (ii) one or more preset thresholds, or using (i) the at least one value, (ii) the formula including the explanatory variable to be substituted with the at least one value, and (iii) one or more preset thresholds. The categories may include (i) a category to which a subject with a high risk of developing the inflammation after parturition (for example, a subject having the blood concentration of haptoglobin after parturition equal to or higher than the criterion value (for example, 800 µg/mL)) belongs and (ii) a category to which a subject with a low risk of developing the inflammation after parturition (for example, a subject having the blood concentration of haptoglobin after parturition lower than the criterion value (for example, 800 µg/mL)) belongs. The categories may include (i) the category to which a subject with a high risk of developing the inflammation after parturition belongs, (ii) the category to which a subject with a low risk of developing the inflammation after parturition belongs, and (iii) a category to which a subject with an intermediate risk of developing the inflammation after parturition belongs.

For example, the blood concentration value of haptoglobin in blood of the subject to be evaluated after parturition may be estimated using (i) the at least one value of the concentration values of the 25 kinds of amino acids, the test values of the 37 kinds of biochemistries, and the measurement values of the four kinds of measurement items and (ii) the one or more preset thresholds or using (i) the at least one value, (ii) the formula including the explanatory variable to be substituted with the at least one value, and (iii) the one or more preset thresholds.

The concentration value, the test value, the measurement value, or the value of the formula may be converted by the predetermined method, and the subject to be evaluated may be classified into any one of the categories using the converted value.

As an index indicating that the inflammation is in the predetermined state, for example, a state where the blood concentration of haptoglobin exceeds a criterion value, as well as d-ROMs as an index of oxidative stress, BAP as an index of antioxidant capacity, IL-1β, serum albumin A, LBP, and a somatic cell count (SCC) in milk may be used.

As for the formula used for the evaluation, the form of the formula is not specifically designated, however, for example, may be the following forms.
- linear model such as multiple regression equation, linear discriminant, principal component analysis, and canonical discriminant analysis that are based on the least-squares method;
- generalized linear model such as logistic regression and Cox regression that are based on the maximum likelihood method;
- generalized linear mixed model considering random effects due to individual differences, facility differences, and other factors in addition to the generalized linear model;
- expression generated by cluster analysis, such as the K-means method, k-nearest neighbor method, and hierarchical cluster analysis;
- expression generated based on the Bayesian statistics such as the Markov chain Monte Carlo (MCMC), the Bayesian network, the Bayesian hierarchical method, and Gaussian process models;
- expression generated by class classification such as support vector machine and decision tree;
- expression generated by ensemble learning models that combine multiple models such as RandomForest, GBDT, lightGBM, and ExtraTrees;
- expression generated based on neural network structures such as deep learning models, Self-Attention, and Transformer;
- expression generated by a method such as fractional expression that does not belong to the above-cited categories; and
- expression represented as, for example, the summation of expressions of different forms

The formula used for the evaluation may be prepared by a method described in WO 2004/052191 that is an international application filed by the present applicant or by a method described in WO 2006/098192 that is an international application filed by the present applicant. Any formulae obtained by these methods can be preferably used in the evaluation of the state of postpartum inflammation, regardless of the units of the concentration values of the amino acids, the test values of the biochemistries, and the measurement values of the measurement items in the blood data as input data.

In the multiple regression equation, the multiple logistic regression equation, and the canonical discriminant function, a coefficient and a constant term are added to each explanatory variable, and the coefficient and the constant term may be preferably real numbers, more preferably values in the range of 99% confidence interval for the coefficient and the constant term obtained from data for the various kinds of classifications described above, more preferably values in the range of 95% confidence interval for the coefficient and the constant term obtained from data for the various kinds of classifications described above. The value of each coefficient and the confidence interval thereof may be those multiplied by a real number, and the value of the constant term and the confidence interval thereof may be those having an arbitrary actual constant added or subtracted or those multiplied or divided by an arbitrary actual constant. When an expression such as the logistic regression equation, the linear discriminant, and the multiple regression equation is used for the evaluation, a linear transformation of the expression (addition of a constant and multiplication by a constant) and a monotonic increasing (decreasing) transformation (for example, a logit transformation) of the expression do not alter evaluation performance and thus the evaluation performance of the expression after transformation is equivalent to that before transformation. Therefore, the expression subjected to the transformation may be used for the evaluation.

In the fractional expression, the numerator of the fractional expression is expressed by the sum of the explanatory variables A, B, C, etc. and the denominator of the fractional expression is expressed by the sum of the explanatory variables a, b, c, etc.
The fractional expression also includes the sum of the fractional expressions α, β, γ, etc. (for example, α+β) having such constitution. The fractional expression also includes divided fractional expressions. The explanatory variables used in the numerator or denominator may have suitable coefficients respectively. The explanatory variables used in the numerator or denominator may appear repeatedly. Each fractional expression may have a suitable coefficient. A value of a coefficient for each explanatory variable and a value for a constant term may be any real numbers. In a fractional expression and the one in which explanatory variables in the numerator and explanatory variables in the denominator in the fractional expression are switched with each other (the fractional expression with the switched explanatory variables), the positive and negative signs are generally reversed in correlation with objective variables, but because their correlation with the objective variables is maintained, the evaluation performance of the expressions can be assumed to be equivalent. The fractional expression therefore also includes the fractional expression with the switched explanatory variables.

### 1-2. Proposal of prophylactic measure

A prophylactic measure is proposed for a cow evaluated in the evaluating step as having a high possibility of suffering from the metabolic disease after parturition. The proposal of the prophylactic measure may be performed after only the evaluation is performed. The proposal of the prophylactic measure may be performed after the evaluation of the present application is performed, for example, after the evaluation described in Japanese Patent Application 2023-074785 is performed before the evaluation.

The prophylactic measure may be at least one selected from the group consisting of administration of Rumen Protect amino acid such as AjiPro (registered trademark)-L, administration of a feed additive, administration of a drug, and veterinary diagnosis. For example, those described in International Publication WO 2008/041371 that is an international application by the present applicant can be used as AjiPro (registered trademark)-L. The feed additive may be at least one selected from the group consisting of PH adjusters, ion balance adjusters, mycotoxin adsorbents, propionate analogs such as calcium propionate, vitamins, minerals, amino acids, fatty acids, urea, probiotics, yeasts, enzymes, antibiotics, antioxidants, antibacterial agents, and organic acids.

For the prophylactic measure, statistical causal inference may be performed to infer a cause of disease for a cow evaluated as having a possibility of suffering from the metabolic disease, and a prophylactic measure corresponding to the cause may be proposed. For the animals evaluated as having a high risk, the prophylactic measure may be proposed by veterinary individual handling.

### Second Embodiment

### 2-1. Outline of second embodiment

Here, outlines of the second embodiment will be described in detail with reference to FIG. 2. FIG. 2 is a principle configurational diagram showing a basic principle of the second embodiment. In the description of the present second embodiment, description duplicating that of the first embodiment is sometimes omitted. In particular, herein, when the state of postpartum inflammation is evaluated, a case of using the value of the formula or the converted value of the value of the formula is described as one example. However, for example, the at least one value of the concentration values of the 25 kinds of amino acids, the test values of the 37 kinds of biochemistries, and the measurement values of the four kinds of measurement items or the converted value of the value may be used.

A control device evaluates the state of postpartum inflammation of the subject to be evaluated by calculating the value of the formula using (i) the at least one value included in the blood data obtained in advance including the at least one value of the concentration values of the 25 kinds of amino acids in blood of the ruminant as the subject to be evaluated before parturition, the test values of the 37 kinds of biochemistries in the blood, and the measurement values of the four kinds of measurement items obtained from the blood and (ii) the formula stored in advance in a memory device including the explanatory variable to be substituted with the at least one value (step S21).

In this way, reliable information on the state of postpartum inflammation can be provided before parturition.

The formula used at step S21 may be generated based on the formula-preparing processing (step 1 to step 4) described below. Here, the summary of the formula-preparing processing is described.

First, the control device prepares a candidate formula (e.g., y=a1x1+a2x2+...+anxn, y: index data, xi: blood data, ai: constant, i=1,2, ..., n), based on a predetermined formula-preparing method from index state information (data such as missing values and outliers may be removed from the index state information in advance) previously stored in the memory device (step 1). The index state information may include blood data on the at least one value of the concentration values of the 25 kinds of amino acids in blood before parturition, the test values of the 37 kinds of biochemistries in the blood, and the measurement values of the four kinds of measurement items obtained from the blood, and index data (e.g. concentration value of haptoglobin in blood after parturition) on the state of postpartum inflammation.

At step 1, a plurality of the candidate formulae may be prepared from the index state information by using a plurality of the different formula-preparing methods (including those for multivariate analysis such as the principal component analysis, the discriminant analysis, the support vector machine, the multiple regression analysis, the Cox regression analysis, the logistic regression analysis, the K-means method, the cluster analysis, the decision tree, the k-nearest neighbor method, the Gaussian process model, the RandomForest, the GBDT, the ExtraTrees, and the neural network). Specifically, a plurality of groups of the candidate formulae may be prepared simultaneously and concurrently by using a plurality of different algorithms with the index state information which is multivariate data composed of the blood data before parturition and the index data after parturition obtained from a large number of ruminants. For example, the two different candidate formulae may be formed by performing the discriminant analysis and the logistic regression analysis simultaneously with the different algorithms. Alternatively, the candidate formula may be formed by converting the index state information with the candidate formula prepared by performing the principal component analysis and then performing the discriminant analysis of the converted index state information. In this way, it is possible to finally prepare the most suitable formula for the evaluation.

The candidate formula prepared by the principal component analysis is a linear expression including each explanatory variable maximizing the variance of all blood data. The candidate formula prepared by the discriminant analysis is a high-powered expression (including exponential and logarithmic expressions) including each explanatory variable minimizing the ratio of the sum of the variances in respective groups to the variance of all blood data. The candidate formula prepared by using the support vector machine is a high-powered expression (including kernel function) including each explanatory variable maximizing the boundary between groups. The candidate formula prepared by using the multiple regression analysis is a high-powered expression including each explanatory variable minimizing the sum of the distances from all blood data. The candidate formula prepared by using the Cox regression analysis is a linear model including a logarithmic hazard ratio, and is a linear expression including each explanatory variable with a coefficient thereof maximizing the likelihood of the linear model. The candidate formula prepared by using the logistic regression analysis is a linear model expressing logarithmic odds of probability, and a linear expression including each explanatory variable maximizing the likelihood of the probability. The K-means method is a method of searching k pieces of neighboring blood data in various groups, designating the group containing the greatest number of the neighboring points as its data-belonging group, and selecting the explanatory variable that makes the group to which input blood data belong agree well with the designated group. The cluster analysis is a method of clustering (grouping) the points closest in entire blood data. The decision tree is a method of ordering explanatory variables and predicting the group of blood data from the pattern possibly held by the higher-ordered explanatory variable.

Returning to the description of the formula-preparing processing, the control device verifies (mutually verifies) the candidate formula prepared at step 1 based on a particular verifying method (step 2). The verification of the candidate formula is performed for each candidate formula prepared at step 1. At step 2, at least one of indexes such as discrimination rate, sensitivity, specificity, information criterion (AIC (Akaike Information Criterition), BIC (Bayesian Information Criterition)), and ROC_AUC (area under the curve in a receiver operating characteristic curve) of the candidate formula may be verified by at least one of methods such as bootstrap method, holdout method, N-fold method, and leave-one-out method. In this way, it is possible to prepare the candidate formula higher in predictability or robustness, by taking the index state information and the evaluation condition into consideration.

In the present description, the discrimination rate is a rate in which a subject to be evaluated whose true state is negative (for example, a subject with no inflammation after parturition) is correctly evaluated as being negative by the evaluation method according to the present embodiment and a subject to be evaluated whose true state is positive (for example, a subject with inflammation after parturition) is correctly evaluated as being positive by the evaluation method according to the present embodiment. In the present description, the sensitivity is a rate in which a subject to be evaluated whose true state is positive is correctly evaluated as being positive by the evaluation method according to the present embodiment. In the present description, the specificity is a rate in which a subject to be evaluated whose true state is negative is correctly evaluated as being negative by the evaluation method according to the present embodiment. The Akaike information criterion is a criterion representing how observation data agrees with a statistical model, for example, in the regression analysis, and it is determined that the model in which the value defined by "-2×(maximum log-likelihood of statistical model)+(the number of free parameters of statistical model)×ln(sample size)" (the model with fewer parameters) is smallest is the best. The ROC_AUC (the area under the receiver operating characteristics curve) is defined as the area under the receiver operating characteristics curve (ROC) created by plotting (x, y)=(1-specificity, sensitivity) on two-dimensional coordinates. The value of ROC_AUC is 1 in perfect discrimination, and the closer this value is to 1, the higher the discriminative performance. The predictability is the average of discrimination rates, sensitivities, or specificities obtained by repeating the validation of the candidate formula. The robustness refers to the variance of discrimination rates, sensitivities, or specificities obtained by repeating the validation of the candidate formula.

Returning to the description of the formula-preparing processing, the control device selects a combination of the blood data contained in the index state information used in preparing the candidate formula, by selecting an explanatory variable of the candidate formula based on a predetermined explanatory variable-selecting method (step 3). At step 3, the selection of the explanatory variable may be performed on each candidate formula prepared at step 1. In this way, it is possible to select the explanatory variable of the candidate formula properly. Step 1 is executed once again by using the index state information including the blood data selected at step 3. At step 3, the explanatory variable of the candidate formula may be selected based on at least one of stepwise method, best path method, neighborhood search method, genetic algorithm, and multi-objective optimization, from the verification result obtained at step 2. The best path method is a method of selecting an explanatory variable by sequentially reducing the number of explanatory variables in the candidate formula one by one and optimizing the evaluation index given by the candidate formula. The multi-objective optimization is a method that simultaneously optimizes two or more evaluation indices (for example, the number of features, training performance, validation performance, or computation time).

Returning to the description of the formula-preparing processing, the control device prepares the formula used for the evaluation, by repeatedly performing steps 1, 2 and 3, and selecting the candidate formula used for the evaluation from the candidate formulae, based on the thus accumulated verification results (step 4). For example, the selection of a candidate formula includes a case where the optimal or multi-objective optimal candidate formula is selected from among candidate formulae prepared by the same formula-preparing method, and a case where the optimal or multi-objective optimal candidate formula is selected from among all candidate formulae.

As described above, in the formula-preparing processing, the optimal formula for evaluating the state of inflammation after parturition can be prepared by performing the preparation of the candidate formulae, the verification of the candidate formulae, and the selection of the explanatory variables in the candidate formulae in a series of steps in an organized (systematic) manner. In other words, in the formula-preparing processing, the concentration values of the 25 kinds of amino acids, the test values of the 37 kinds of biochemistries, and the measurement values of the four kinds of measurement items are used in multivariate statistical analysis, and for selecting the optimum and robust combination of the explanatory variables, the explanatory variable-selecting method is combined with cross-validation to extract the formula having high evaluation performance.

### 2-2. System configuration

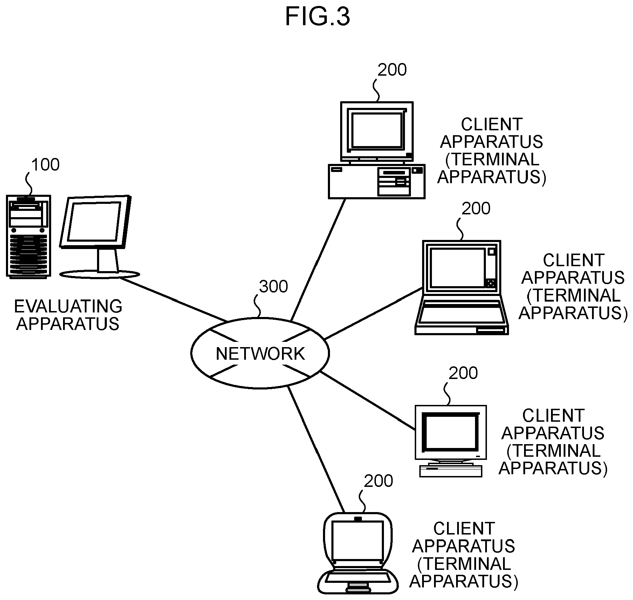

Hereinafter, the configuration of the evaluating system according to the second embodiment (hereinafter, referred to sometimes as the present system) will be described with reference to FIGS. 3 to 13. This system is merely one example, and the present invention is not limited thereto. In particular, herein, when the state of postpartum inflammation is evaluated, a case of using the value of the formula or the converted value of the value of the formula is described as one example. However, for example, the at least one value of the concentration values of the 25 kinds of amino acids, the test values of the 37 kinds of biochemistries, and the measurement values of the four kinds of measurement items or the converted value of the value may be used.

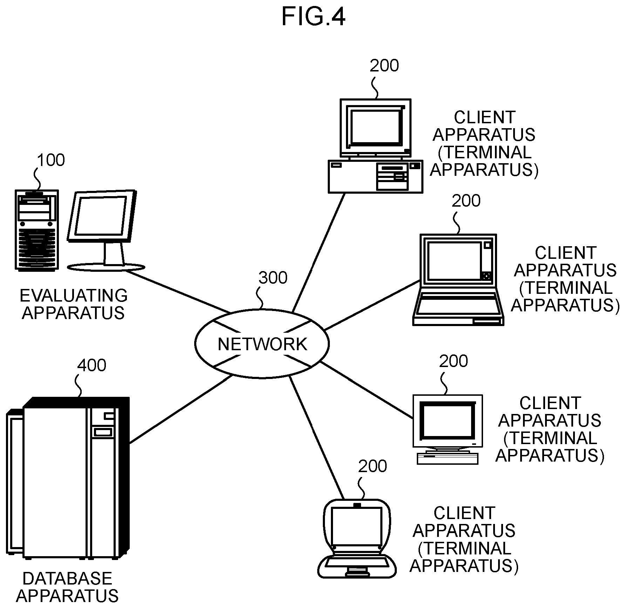

First, an entire configuration of the present system will be described with reference to FIGS. 3 and 4. FIG. 3 is a diagram showing an example of the entire configuration of the present system. FIG. 4 is a diagram showing another example of the entire configuration of the present system. As shown in FIG. 3, the present system is constituted in which the evaluating apparatus 100 that evaluates the state of postpartum inflammation and the client apparatus 200 (corresponding to the terminal apparatus of the present invention) that provides the blood data are communicatively connected to each other via a network 300.

In the present system, the client apparatus 200 that provides data for use in the evaluation and the client apparatus 200 that receives the evaluation result may be different. In the present system as shown in FIG. 4, in addition to the evaluating apparatus 100 and the client apparatus 200, the database apparatus 400 storing, for example, the index state information used in preparing the formula in the evaluating apparatus 100 and the formula used for the evaluation may be communicatively connected via the network 300.

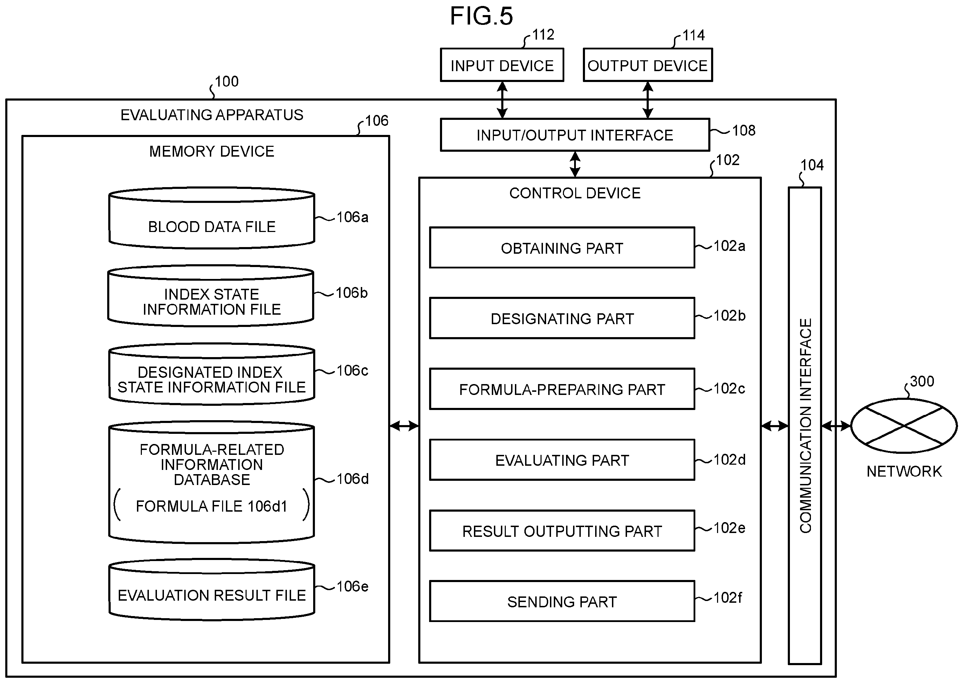

Now, the configuration of the evaluating apparatus 100 in the present system will be described with reference to FIGS. 5 to 11. FIG. 5 is a block diagram showing an example of the configuration of the evaluating apparatus 100 in the present system, showing conceptually only the region relevant to the present invention.

The evaluating apparatus 100 includes (i) a control device 102, such as CPU (Central Processing Unit), that integrally controls the evaluating apparatus, (ii) a communication interface 104 that connects the evaluating apparatus to the network 300 communicatively via communication apparatuses such as a router and wired or wireless communication lines such as a private line, (iii) a memory device 106 that stores various databases, tables, files and others, and (iv) an input/output interface 108 connected to an input device 112 and an output device 114, and these parts are connected to each other communicatively via any communication channel. The evaluating apparatus 100 may be configured in the same housing as a variety of analyzing apparatuses (e.g., amino acid and biochemistry analyzing apparatus). For example, the evaluating apparatus 100 may be a compact analyzing device including components (hardware and software) that calculate (measure) the at least one value of the concentration values of the 25 kinds of amino acids, the test values of the 37 kinds of biochemistries, and the measurement values of the four kinds of measurement items and output (e.g., print or display on a monitor) the calculated value, wherein the compact analyzing device further includes the evaluating part 102d described later, and uses the components to output results obtained by the evaluating part 102d.

The communication interface 104 allows communication between the evaluating apparatus 100 and the network 300 (or a communication apparatus such as a router). Thus, the communication interface 104 has a function to communicate data via a communication line with other terminals.

The input/output interface 108 is connected to the input device 112 and the output device 114. A monitor (including a home television), a speaker, or a printer may be used as the output device 114. A keyboard, a mouse, a microphone, or a monitor functioning as a pointing device together with a mouse may be used as the input device 112.

The memory device 106 is a storage means, and examples thereof include a memory apparatus such as RAM (Random Access Memory) and ROM (Read Only Memory), a fixed disk drive such as a hard disk, a flexible disk, and an optical disk. The memory device 106 stores computer programs giving instructions to the CPU for various processings, together with OS (Operating System). As shown in the figure, the memory device 106 stores the blood data file 106a, the index state information file 106b, the designated index state information file 106c, a formula-related information database 106d, and the evaluation result file 106e.

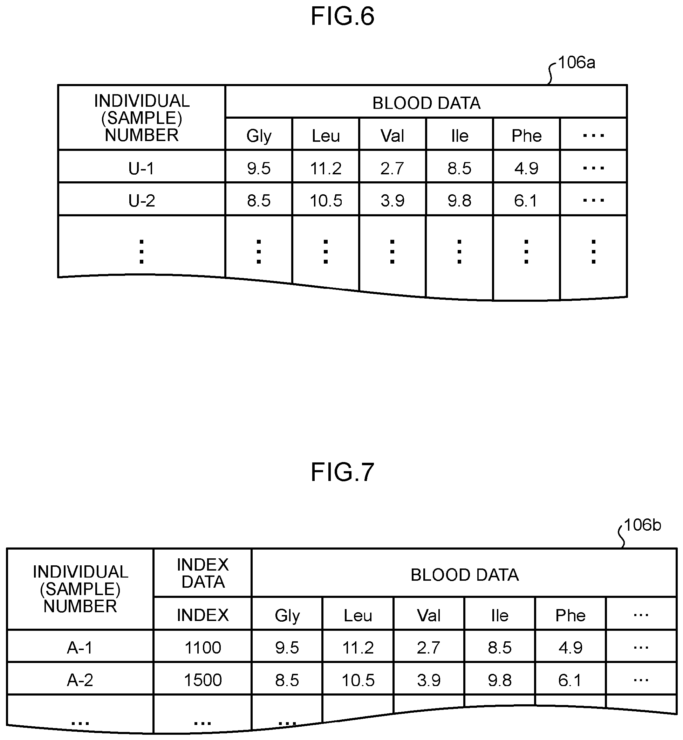

The blood data file 106a stores the blood data on the at least one value of the concentration values of the 25 kinds of amino acids in the blood, the test values of the 37 kinds of biochemistries in the blood, and the measurement values of the four kinds of measurement items obtained from the blood. FIG. 6 is a chart showing an example of information stored in the blood data file 106a. As shown in FIG. 6, the information stored in the blood data file 106a includes an individual number for uniquely identifying an individual (sample) as the subject to be evaluated and the blood data that are correlated to one another. In FIG. 6, the blood data is assumed to be numerical values, i.e., on a continuous scale, but the blood data may be expressed on a nominal scale or an ordinal scale. In the case of the nominal or ordinal scale, any number may be allocated to each state for analysis. The blood data may be combined with the value related to the factors described above having an impact on the development of inflammation.

Returning to FIG. 5, the index state information file 106b stores the index state information used in preparing the formula. FIG. 7 is a chart showing an example of information stored in the index state information file 106b. As shown in FIG. 7, the information stored in the index state information file 106b includes the individual number, the index data (T) on inflammation after parturition such as concentration value of haptoglobin in blood after partirition, and the blood data that are correlated to one another. In FIG. 7, the index data and the blood data are assumed to be numerical values, i.e., on a continuous scale, but the index data and the blood data may be expressed on a nominal scale or an ordinal scale. If the index data and blood data are on a nominal or ordinal scale, arbitrary numerical values assigned to each state may be used in various processes, such as evaluations or calculations of formula values.

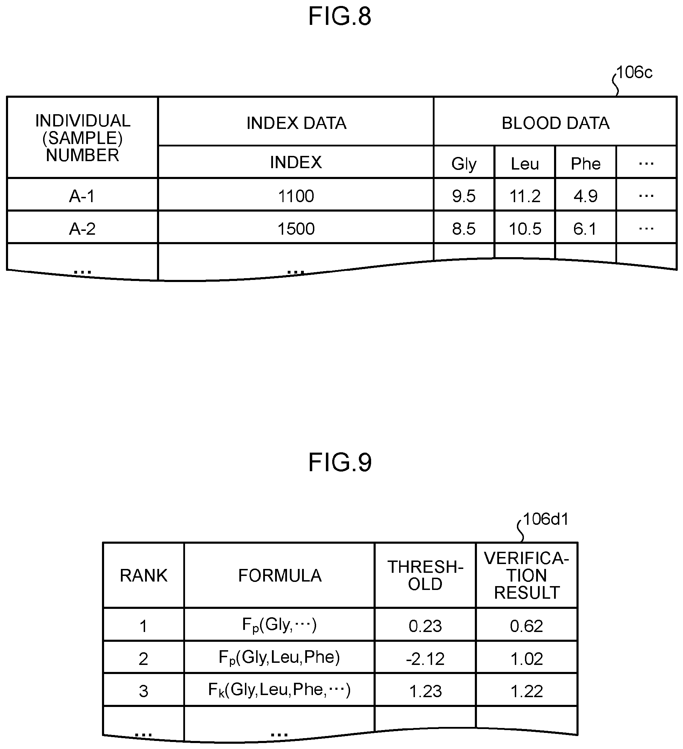

Returning to FIG. 5, the designated index state information file 106c stores the index state information designated in a designating part 102b described below. FIG. 8 is a chart showing an example of information stored in the designated index state information file 106c. As shown in FIG. 8, the information stored in the designated index state information file 106c includes the individual number, the designated index data, and the designated blood data that are correlated to one another.

Returning to FIG. 5, the formula-related information database 106d is composed of the formula file 106d1 storing the formula prepared in a formula-preparing part 102c described below. The formula file 106d1 stores the formulae used for the evaluation. FIG. 9 is a chart showing an example of information stored in the formula file 106d1. As shown in FIG. 9, the information stored in the formula file 106d1 includes a rank, the formula (e.g., Fp (Gly, ...), Fp (Gly, Leu, Phe), Fk (Gly, Leu, Phe, ...) in FIG. 9), a threshold corresponding to each formula-preparing method, and the verification result of each formula (e.g., the value of each formula) that are correlated to one another.

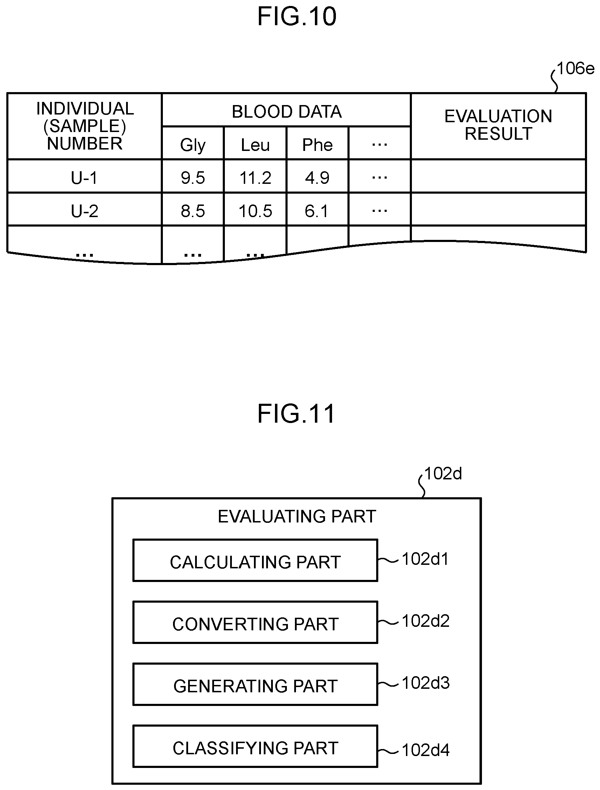

Returning to FIG. 5, the evaluation result file 106e stores the evaluation results obtained in the evaluating part 102d described below. FIG. 10 is a chart showing an example of information stored in the evaluation result file 106e. The information stored in the evaluation result file 106e includes the individual number for uniquely identifying the individual (sample) as the subject to be evaluated, the previously obtained blood data of the individual, and the evaluation result on the state of inflammation after parturition (for example, the value of the formula calculated by a calculating part 102d1 described below, the converted value obtained by a converting part 102d2 described below, the positional information generated by a generating part 102d3 described below, or the classification result obtained by a classifying part 102d4 described below) that are correlated to one another.

Returning to FIG. 5, the control device 102 has an internal memory storing, for example, control programs such as OS (Operating System), programs for various processing procedures, and other needed data, and performs various information processings according to these programs. As shown in the figure, the control device 102 includes mainly an obtaining part 102a, the designating part 102b, the formula-preparing part 102c, the evaluating part 102d, a result outputting part 102e, and a sending part 102f. The control device 102 performs data processings, such as removal of data including missing values, removal of data including many outliers, and removal of explanatory variables for the data including missing values, in the index state information transmitted from the database apparatus 400 and in the blood data transmitted from the client apparatus 200.

The obtaining part 102a obtains information (specifically, the blood data, the index state information, and the formula, etc.). For example, the obtaining part 102a may obtain the information by receiving, via the network 300, information (specifically, the blood data, the index state information, and the formula, etc.) transmitted from the client apparatus 200 and the database apparatus 400. The obtaining part 102a may receive data used for evaluation transmitted from a client apparatus 200 different from the client apparatus 200 to which the evaluation results are transmitted. For example, if the evaluating apparatus 100 includes a mechanism (including hardware and software) for reading information recorded on a recording medium, the obtaining part 102a may obtain the information (specifically, the blood data, the index state information, and the formula, etc.) by reading the information recorded on the recording medium via the mechanism. The designating part 102b designates objective index data and objective blood data in preparing the formula.

The formula-preparing part 102c generates the formula based on the index state information obtained by the obtaining part 102a or the index state information designated in the designating part 102b. If the formulae are stored previously in a predetermined region of the memory device 106, the formula-preparing part 102c may generate the formula by selecting the desired formula out of the memory device 106. Alternatively, the formula-preparing part 102c may generate the formula by selecting and downloading the desired formula from another computer apparatus (e.g., the database apparatus 400) in which the formulae are previously stored.

The evaluating part 102d evaluates the state of inflammation in the individual after parturition by calculating the value of the formula using (i) the previously obtained formula (for example, the formula prepared by the formula-preparing part 102c or the formula obtained by the obtaining part 102a) and (ii) the at least one value of the concentration values of the 25 kinds of amino acids, the test values of the 37 kinds of biochemistries, and the measurement values of the four kinds of measurement items included in the blood data obtained by the obtaining part 102a. The evaluating part 102d may evaluate the state of inflammation in the individual after parturition using (i) the at least one value of the concentration values of the 25 kinds of amino acids, the test values of the 37 kinds of biochemistries, and the measurement values of the four kinds of measurement items or (ii) the converted value thereof.

Hereinafter, a configuration of the evaluating part 102d will be described with reference to FIG. 11. FIG. 11 is a block diagram showing the configuration of the evaluating part 102d, and only a part in the configuration related to the present invention is shown conceptually. The evaluating part 102d includes the calculating part 102d1, the converting part 102d2, the generating part 102d3, and the classifying part 102d4, additionally.

The calculating part 102d1 calculates the value of the formula using (i) the at least one value of the concentration values of the 25 kinds of amino acids, the test values of the 37 kinds of biochemistries, and the measurement values of the four kinds of measurement items and (ii) the formula including an explanatory variable to be substituted with the at least one value. The evaluating part 102d may store the value of the formula calculated by the calculating part 102d1 as the evaluation result in a predetermined region of the evaluation result file 106e.

The converting part 102d2 converts the value of the formula calculated by the calculating part 102d1, for example, by the conversion method described above. The converting part 102d2 may convert the at least one value of the concentration values of the 25 kinds of amino acids, the test values of the 37 kinds of biochemistries, and the measurement values of the four kinds of measurement items included in the blood data, for example, by the conversion method described above. The evaluating part 102d may store the converted value by the converting part 102d2 as the evaluation result in a predetermined region of the evaluation result file 106e.

The generating part 102d3 generates the positional information about the position of the predetermined mark on the predetermined scale visually presented on the display device such as a monitor or the physical medium such as paper, using the value of the formula calculated by the calculating part 102d1 or the converted value by the converting part 102d2 (the concentration value or test value, or the converted value of the concentration value or test value may be used as well). The evaluating part 102d may store the positional information generated by the generating part 102d3 as the evaluation result in a predetermined region of the evaluation result file 106e.

The classifying part 102d4 classifies the individual into any one of the categories defined at least considering the degree of the risk of developing inflammation after parturition, using the value of the formula calculated by the calculating part 102d1 or the converted value by the converting part 102d2 (the concentration value or the test value, or the converted value of the concentration value or test value may be used as well).

The result outputting part 102e outputs, into the output device 114, for example, the processing results in each processing part in the control device 102 (including the evaluation results obtained by the evaluating part 102d).

The sending part 102f transmits the evaluation results to the client apparatus 200 that is a sender of the blood data of the individual, and transmits the formulae prepared in the evaluating apparatus 100 and the evaluation results to the database apparatus 400. The sending part 102f may transmit the evaluation result to the client apparatus 200 different from the client apparatus 200 that from which data for use in the evaluation is transmitted.

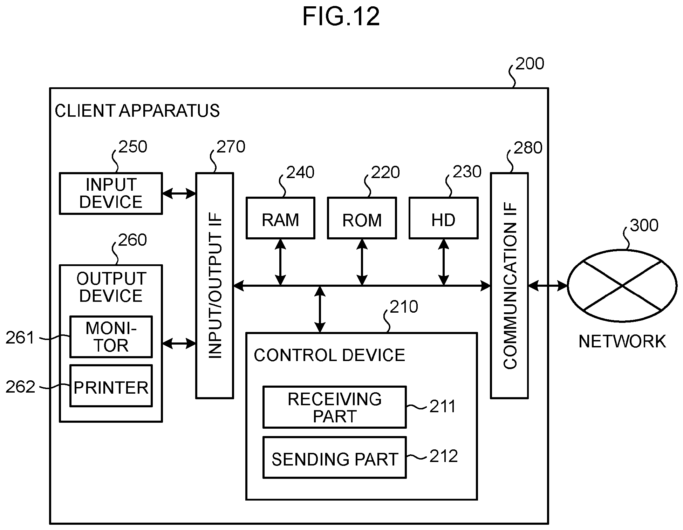

Hereinafter, a configuration of the client apparatus 200 in the present system will be described with reference to FIG. 12. FIG. 12 is a block diagram showing an example of the configuration of the client apparatus 200 in the present system, and only the part in the configuration relevant to the present invention is shown conceptually.

The client apparatus 200 includes a control device 210, ROM 220, a HD (Hard Disk) 230, a RAM 240, an input device 250, an output device 260, an input/output IF 270, and a communication IF 280 that are connected communicatively to one another through a communication channel. The client apparatus 200 may be realized based on an information processing apparatus (for example, an information processing terminal such as a known personal computer, a workstation, a family computer, Internet TV (Television), PHS (Personal Handyphone System) terminal, a mobile phone terminal, a mobile unit communication terminal, or PDA (Personal Digital Assistant)) connected as needed with peripheral devices such as a printer, a monitor, and an image scanner.

The input device 250 is, for example, a keyboard, a mouse, or a microphone. The monitor 261 described below also functions as a pointing device together with a mouse. The output device 260 is an output means for outputting information received via the communication IF 280, and includes the monitor 261 (including home television) and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as a router) communicatively. In other words, the client apparatus 200 is connected to the network 300 via a communication apparatus such as a modem, TA (Terminal Adapter) or a router, and a telephone line, or via a private line. In this way, the client apparatus 200 can access to the evaluating apparatus 100 by using a particular protocol.

The control device 210 has a receiving part 211 and a sending part 212. The receiving part 211 receives various kinds of information such as the evaluation results transmitted from the evaluating apparatus 100, via the communication IF 280. The sending part 212 sends various kinds of information such as the blood data of the individual, via the communication IF 280, to the evaluating apparatus 100.

All or a part of processings of the control device 210 in the client apparatus 200 may be performed by CPU and programs read and executed by the CPU. Computer programs for giving instructions to the CPU and executing various processings together with the OS (Operating System) are recorded in the ROM 220 or HD 230. The computer programs, which are executed as they are loaded in the RAM 240, constitute the control device 210 with the CPU. The computer programs may be stored in application program servers connected via any network to the client apparatus 200, and the client apparatus 200 may download all or a part of them as needed. All or any part of processings of the control device 210 may be realized by hardware such as wired-logic.

The control device 210 may include an evaluating part 210a (including a calculating part 210a1, a converting part 210a2, a generating part 210a3, and a classifying part 210a4) having the same functions as the functions of the evaluating part 102d in the evaluating apparatus 100. When the control device 210 includes the evaluating part 210a, the evaluating part 210a may convert the value of the formula (the concentration value or the test value may be used as well) in the converting part 210a2, generate the positional information corresponding to the value of the formula or the converted value (the concentration value or the test value, or the converted value of the concentration value or the test value may be used as well) in the generating part 210a3, and classify the individual into any one of the categories using the value of the formula or the converted value (the concentration value or the test value, or the converted value of the concentration value or the test value may be used as well) in the classifying part 210a4, in accordance with information included in the evaluation result transmitted from the evaluating apparatus 100.

Hereinafter, the network 300 in the present system will be described with reference to FIGS. 3 and 4. The network 300 has a function to connect the evaluating apparatus 100, the client apparatuses 200, and the database apparatus 400 mutually, communicatively to one another, and is for example the Internet, an intranet, or LAN (Local Area Network (including both wired and wireless)). The network 300 may be VAN (Value Added Network), a personal computer communication network, a public telephone network (including both analog and digital), a leased line network (including both analog and digital), CATV (Community Antenna Television) network, a portable switched network or a portable packet-switched network (including IMT2000 (International Mobile Telecommunication 2000) system, GSM (registered trademark) (Global System for Mobile Communications) system, PDC (Personal Digital Cellular)/PDC-P system, and the like), a wireless calling network, a local wireless network such as Bluetooth (registered trademark), PHS network, a satellite communication network (including CS (Communication Satellite), BS (Broadcasting Satellite), ISDB (Integrated Services Digital Broadcasting), and the like), or the like.

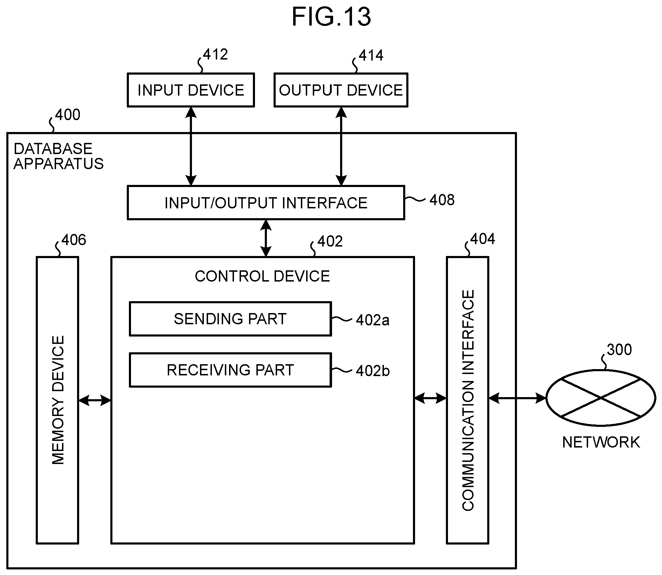

Hereinafter, the configuration of the database apparatus 400 in the present system will be described with reference to FIG. 13. FIG. 13 is a block diagram showing an example of the configuration of the database apparatus 400 in the present system, showing conceptually only the region relevant to the present invention.

The database apparatus 400 has functions to store, for example, the index state information used in preparing the formulae in the evaluating apparatus 100 or the database apparatus, the evaluation formulae prepared in the evaluating apparatus 100, and the evaluation results obtained in the evaluating apparatus 100. As shown in FIG. 13, the database apparatus 400 includes (i) a control device 402, such as CPU, that integrally controls the database apparatus, (ii) a communication interface 404 connecting the database apparatus to the network 300 communicatively via communication apparatuses such as a router and wired or wireless communication circuits such as a private line, (iii) a memory device 406 storing various databases, tables, files, and others, and (iv) an input/output interface 408 connected to an input device 412 and an output device 414, and these parts are connected communicatively to each other via any communication channel.

The memory device 406 is a storage means, and, examples thereof include a memory apparatus such as RAM or ROM, a fixed disk drive such as a hard disk, a flexible disk, and an optical disk. The memory device 406 stores, for example, various programs used in various processings. The communication interface 404 allows communication between the database apparatus 400 and the network 300 (or a communication apparatus such as a router). Thus, the communication interface 404 has a function to communicate data via a communication line with other terminals. The input/output interface 408 is connected to the input device 412 and the output device 414. A monitor (including a home television), a speaker, or a printer may be used as the output device 414. A keyboard, a mouse, a microphone, or a monitor functioning as a pointing device together with a mouse may be used as the input device 412.

The control device 402 has an internal memory storing, for example, control programs such as OS (Operating System), programs for various processing procedures, and other needed data, and performs various information processings according to these programs. As shown in the figure, the control device 402 includes mainly a sending part 402a and a receiving part 402b. The sending part 402a transmits various kinds of information such as the index state information and the formulae to the evaluating apparatus 100. The receiving part 402b receives various kinds of information such as the formulae and the evaluation results transmitted from the evaluating apparatus 100.

In the present description, the evaluating apparatus 100 executes the obtaining of the blood data, the calculation of the value of the formula, the classification of the individual into the category, and the transmission of the evaluation result, while the client apparatus 200 executes the reception of the evaluation result, described as an example. However, when the client apparatus 200 includes the evaluating part 210a, the evaluating apparatus 100 only has to execute the calculation of the value of the formula. For example, the conversion of the value of the formula, the generation of the positional information, and the classification of the individual into the category may be appropriately shared between the evaluating apparatus 100 and the client apparatus 200.

For example, when the client apparatus 200 receives the value of the formula from the evaluating apparatus 100, the evaluating part 210a may convert the value of the formula in the converting part 210a2, generate the positional information corresponding to the value of the formula or the converted value in the generating part 210a3, and classify the individual into any one of the categories using the value of the formula or the converted value in the classifying part 210a4.

When the client apparatus 200 receives the converted value from the evaluating apparatus 100, the evaluating part 210a may generate the positional information corresponding to the converted value in the generating part 210a3, and classify the individual into any one of the categories using the converted value in the classifying part 210a4.

When the client apparatus 200 receives the value of the formula or the converted value and the positional information from the evaluating apparatus 100, the evaluating part 210a may classify the individual into any one of the categories using the value of the formula or the converted value in the classifying part 210a4.

### 2-3. Other embodiments

In addition to the second embodiment described above, the evaluating apparatus, the calculating apparatus, the evaluating method, the calculating method, the evaluating program, the calculating program, the recording medium, the evaluating system, and the terminal apparatus according to the present invention can be practiced in various different embodiments within the technological scope of the claims.

Of the processings described in the second embodiment, all or a part of the processings described as automatically performed ones may be manually performed, or all or a part of the processings described as manually performed ones may be also automatically performed by known methods.

In addition, the processing procedures, the control procedures, the specific names, the information including parameters such as registered data of various processings and retrieval conditions, the screen examples, and the database configuration shown in the description and the drawings may be arbitrarily modified unless otherwise specified.

The components of the evaluating apparatus 100 shown in the figures are functionally conceptual and therefore not be physically configured as shown in the figures.

For example, for the operational functions provided in the evaluating apparatus 100, in particular, for the operational functions performed in the control device 102, all or part thereof may be implemented by the CPU (Central Processing Unit) and programs interpreted and executed in the CPU, or may be implemented by wired-logic hardware. The program is recorded in a non-transitory tangible computer-readable recording medium including programmed instructions for making an information processing apparatus execute the evaluating method or the calculating method according to the present invention, and is mechanically read as needed by the evaluating apparatus 100. More specifically, computer programs to give instructions to the CPU in cooperation with the OS (operating system) to perform various processes are recorded in the memory device 106 such as ROM or a HDD (hard disk drive). The computer programs are executed by being loaded to RAM, and form the control unit in cooperation with the CPU.

The computer programs may be stored in an application program server connected to the evaluating apparatus 100 via an arbitrary network, and all or part thereof can be downloaded as necessary.

The evaluating program or the calculating program according to the present invention may be stored in the non-transitory tangible computer-readable recording medium, or can be configured as a program product. The "recording medium" mentioned here includes any "portable physical medium" such as a memory card, a USB (universal serial bus) memory, an SD (secure digital) card, a flexible disk, a magneto-optical disc, ROM, EPROM (erasable programmable read only memory), EEPROM (registered trademark) (electronically erasable and programmable read only memory), CD-ROM (compact disc read only memory), MO (magneto-optical disk), DVD (digital versatile disc), and Blu-ray (registered trademark) Disc.

The "program" mentioned here is a data processing method described in an arbitrary language or description method, and therefore any form such as a source code and a binary code is acceptable. The "program" is not necessarily limited to a program configured as a single unit, and, therefore, includes those dispersively configured as a plurality of modules and libraries and those in which the function of the program is achieved in cooperation with separate programs represented as OS (operating system). Any known configuration and procedures can be used as a specific configuration and reading procedure to read a recording medium by each apparatus shown in the embodiments, an installation procedure after the reading, and the like.

The various databases and the like stored in the memory device 106 is a storage unit such as a memory device such as RAM and ROM, a fixed disk drive such as a hard disk, a flexible disk, or an optical disc. The memory device 106 stores various programs, tables, databases, files for Web (World Wide Web) pages, and the like used to perform various processes and to provide Web sites.

The evaluating apparatus 100 may be configured as an information processing apparatus such as known personal computer and work station, or may be configured as the information processing apparatus connected to an arbitrary peripheral device. The evaluating apparatus 100 may be provided by installing software (including the programs and the data, etc.) to cause the information processing apparatus to implement the evaluating method or the calculating method according to the present invention.

Furthermore, a specific configuration of dispersion or integration of the apparatuses is not limited to the shown one. The apparatuses can be configured by functionally or physically dispersing or integrating all or part of the apparatuses in arbitrary units according to various types of additions or the like or according to functional loads. In other words, the embodiments may be implemented in arbitrary combinations thereof or an embodiment may be selectively implemented.

### Example 1

In 2019, at the University of Minnesota farm, a total of 1,038 blood samples were collected from a total of 519 female Holstein dairy cows 21 days before the expected date of parturition and 7 days after the date of parturition. The 519 dairy cows include 210 first-calf heifers (nulliparous cows) having no experience of parturition other than the present parturition and 309 parous cows having an experience of parturition in addition to the present parturition.

The blood samples collected 21 days before the expected date of parturition were measured to determine the blood concentrations of 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of 29 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), Glob (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood samples collected 7 days after the date of parturition were measured to determine the blood concentration of BHBA. The blood concentrations of the amino acids were measured by the measuring method (A) or (B).

The presence or absence of development of metritis was observed for up to 15 days of days in milk (DIM), the presence or absence of development of mastitis was observed for up to 380 days of DIM, and the presence or absence of clinical ketosis was observed for up to 27 days of DIM. For mastitis, individuals diagnosed with mastitis based on clinical findings were classified as individuals with clinical mastitis, and individuals determined as having mastitis based on the somatic cell count (SCC) in milk were classified as individuals with SCC mastitis. SCC is the collective term for leukocytes and shedding epithelial cells in milk. SCC is said to be higher when an individual suffers from mastitis. In the present example, if "SCC≥200,000/mL", the case is determined as having mastitis.

First, the 519 individuals were randomly split 8:2. Data associated with 80% of the individuals (the measured concentrations and observations regarding the presence or absence of development of each disease) was used as training data, and data associated with the remaining 20% of the individuals (the measured concentrations and observations regarding the presence or absence of development of each disease) was used as verification data.

In the training data, the incidence rate of metritis was 15%, the incidence rate of clinical mastitis was 45%, the incidence rate of SCC mastitis was 22%, and the incidence rate of clinical ketosis was 19%. In the verification data, the incidence rate of metritis was 20%, the incidence rate of clinical mastitis was 44%, the incidence rate of SCC mastitis was 28%, and the incidence rate of clinical ketosis was 18%.

In the training data, an index (formula) to predict (discriminate) the presence or absence of each disease was searched for by a stepwise method (minimum AICc, variable increase, and combination of components) provided by statistical analysis software "JMP (registered trademark) Software", using the blood concentrations of the 25 kinds of amino acids 21 days before the expected date of parturition and the concentration values of the 29 kinds of blood chemistry parameters 21 days before the expected date of parturition. The searched formulae were evaluated by the AUC of the ROC curve.

The prediction performance of the formulae was evaluated by fitting the verification data to the formulae searched based on the training data and by the AUC of the ROC curve.

Some of the formulae that showed good prediction performance for the presence or absence of development of each disease are listed below. These formulae are useful in evaluating the presence or absence of development of each disease.
- Prediction formula for metritis=-4.4+0.31*BUN+0.13*Gly-1.91*ASP-0.25*Val+0.44*Ile-0.02*ALP-0.01*TCHO ROC_AUC=0.70 for the prediction formula based on the training data ROC_AUC=0.60 for the prediction formula based on the verification data
- Prediction formula for clinical mastitis=-1+0.26*Tau-0.03*BUN+1.83*3MeHis-0.06*Gly+1.64*Asp-0.13*Glu-0.17*Pro-0.14*Orn+0.23*Lys-0.33*Phe+0.005*TCHO+0.18*TP ROC_AUC=0.65 for the prediction formula based on the training data ROC_AUC=0.58 for the prediction formula based on the verification data
- Prediction formula for clinical mastitis searched by narrowing the training data with DIM≤21=1.49+0.29*Tau+0.35*Arg-0.36*Ala-1.44*Orn+0.42*Lys+1.30*Met ROC_AUC=0.81 for the prediction formula based on the training data ROC_AUC=0.66 for the prediction formula based on the verification data
- Prediction formula for clinical ketosis=3.87-0.59*Arg+0.5*Thr+0.38*Lys-1.04*Met-0.57*Phe-0.02*ALP-0.03*Glc ROC_AUC=0.75 for the prediction formula based on the training data ROC_AUC=0.64 for the prediction formula based on the verification data

### Example 2

At three experiment stations, namely, the University of Florida, the University of Illinois, and a Japanese farm, a total of 210 blood samples were collected from a total of 105 female Holstein dairy cows (all parous cows) 21 days before the expected date of parturition and 7 days after the date of parturition. The 105 parous cows include 48 from the University of Florida, 17 from the University of Illinois, and 40 from the Japanese farm.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of 28 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood samples collected 7 days after the date of parturition were measured to determine the blood concentration of haptoglobin. The blood concentrations of the amino acids were measured by the measuring method (A) or (B). The blood concentration of haptoglobin was measured by a measuring method called enzyme-linked immuno sorbent assay (ELISA).

Individuals with the postpartum haptoglobin concentration lower than 800 µg/ml were classified into a healthy group, and individuals with the postpartum haptoglobin concentration equal to or higher than 800 µg/ml were classified into an inflammatory group. The criterion value of 800 µg/ml was set with reference to "Dubuc et al. 2010. Risk factors for postpartum uterine diseases in dairy cows". Seventeen percent of the 48 cows from the University of Florida were classified in the inflammatory group, 35% of the 17 cows from the University of Illinois were classified in the inflammatory group, and 18% of the 40 cows from the Japanese farm were classified in the inflammatory group.

An index (formula) to predict (discriminate) the inflammatory conditions was searched for by a stepwise method (minimum AICc, variable increase, and combination of components) provided by statistical analysis software "JMP (registered trademark) Software", using the blood concentrations of the 25 kinds of amino acids 21 days before the expected date of parturition and the concentration values of the 28 kinds of blood chemistry parameters 21 days before the expected date of parturition. The searched formulae were evaluated by the AUC of the ROC curve.

Some of the formulae that showed good prediction performance for the inflammatory conditions are listed below. This formula is useful in evaluating the inflammatory conditions (high haptoglobin).
- Prediction formula for inflammatory conditions (high haptoglobin)=15.21+0.3*Ile+0.06*His-1.09*Phe-0.37*Glu-0.03*Gly-0.96*T-Bil-0.03*TCHO ROC_AUC=0.84 for the prediction formula

### Example 3

At a Japanese farm, a total of 80 blood samples were collected from a total of 40 female Holstein dairy cows (all parous cows) 21 days before the expected date of parturition and 7 days after the date of parturition.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of the 28 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood samples collected 7 days after the date of parturition were measured to determine the blood concentrations of haptoglobin, IL-1β, and serum albumin A as well as d-ROMs and BAP values. The blood concentrations of the amino acids were measured by the measuring method (A) or (B). The blood concentrations of haptoglobin, IL-1β, and serum albumin A were measured by a measuring method called enzyme-linked immuno sorbent assay (ELISA). The d-ROMs and BAP values were measured by a measuring method called colorimetric method.

An index (formula) to predict (discriminate) the inflammatory conditions was searched for by a stepwise method (minimum AICc, variable increase, and combination of components) provided by statistical analysis software "JMP (registered trademark) Software", using the blood concentrations of the 25 kinds of amino acids 21 days before the expected date of parturition and the concentration values of the 28 kinds of blood chemistry parameters 21 days before the expected date of parturition. The multiple regression equations prepared were evaluated by the numerical value of the coefficient of determination R2. The results are listed in Table 1.

The IL-1β, the d-ROMs value, and the serum amyloid A had good prediction accuracy with R2 equal to or more than 0.7. On the other hand, the BAP value did not have good prediction accuracy, but when the ratio to the d-ROMs value was used as the objective variable, the prediction accuracy was good.

### [Table 1]

**(Table 1)**

| OBJECTIVE VARIABLE NUMERICAL VALUE | PREDICTION FORMULA | P VALUE | CORRELATION R2 VALUE BETWEEN PREDICTED VALUE AND MEASURED VALUE |
|---|---|---|---|
| IL-1 β | 1 050.3+154.8*TP-245.3*Ca+100.6*P-61*GGT +0.17*LDH-752.3*T-Bil+0.77*CPK-273.7*Mg +14.9*TG-48.86*His+57.4*Ser | <0.0001 | 0.76 |
| SERUM AMYLOIDA | 60.14-4.3*LA-0.15*BAP-39.9*TP+84.4*K+1.48 *AST-0.12*AMY+5.9+Gln-456.5%Asp+36.1%Glu +7.4*Ala | <0.0001 | 0.84 |
| d-ROMs | -364.1+0.4*d-ROMs-0.02*BAP+3.4*Na-1.4*LA +3.8*Thr+5.5*Arg-4.2*Pro-7.2*Tyr-7.9* 3MeHis | <0.0001 | 0.78 |
| BAP | 3108.7+9.9*ALT-1 36.3*Lys+122.7*Thr-124.5 *Phe+224.5*Arg-20.3*Gln-30.3*Ala | <0.0001 | 0.59 |
| d-ROMs/BAP | 465.8+0.21*BAP/d-ROMs-3.1*Na-257* T-Bil +1.1*LA-4.0*Lys+13.1*3MeHis+4.9*Orn | <0.0001 | 0.76 |

### Example 4

At 20 dairy farms, a total of 1524 blood samples were collected from a total of 762 female Holstein dairy cows 21 days before the expected date of parturition and 7 days after the date of parturition. The 762 dairy cows include 99 first-calf heifers (nulliparous cows) having no experience of parturition other than the present parturition and 663 parous cows having an experience of parturition in addition to the present parturition.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of 29 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), Glob (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood samples collected 7 days after the date of parturition were measured to determine the blood concentration of haptoglobin. The blood concentrations of the amino acids were measured by the measuring method (A) or (B). The blood concentration of haptoglobin was measured by a measuring method called enzyme-linked immuno sorbent assay (ELISA).

Of the dataset of the 762 cows, those with missing values, component concentrations outside a quantitative range, or deaths during the study were excluded. For preparation of prediction formulae, two kinds of models, namely, linear regression models and logistic regression models were prepared.

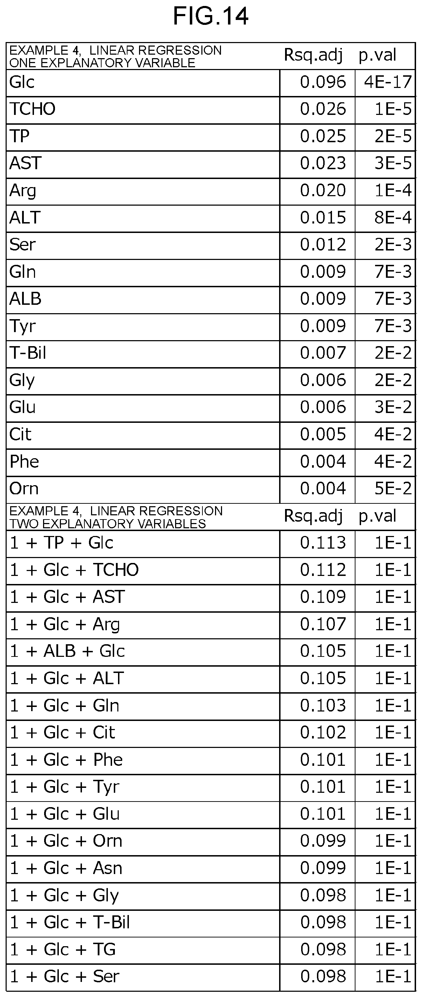

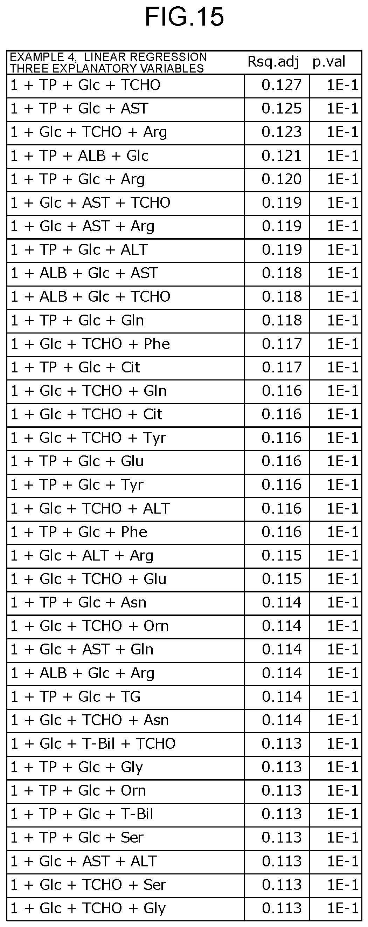

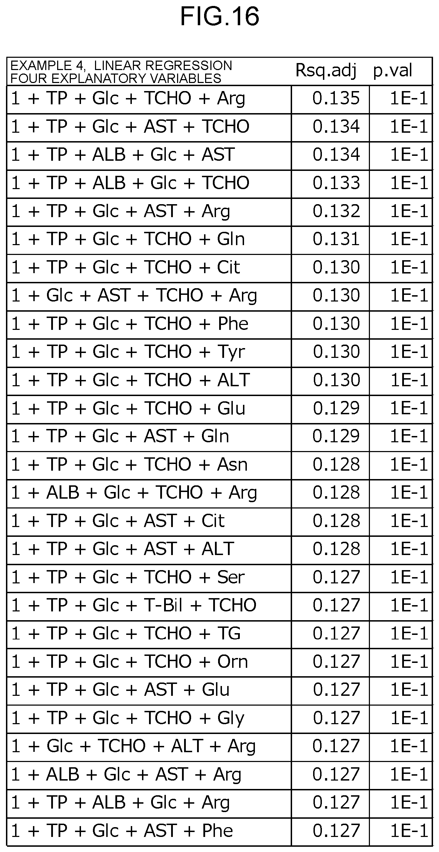

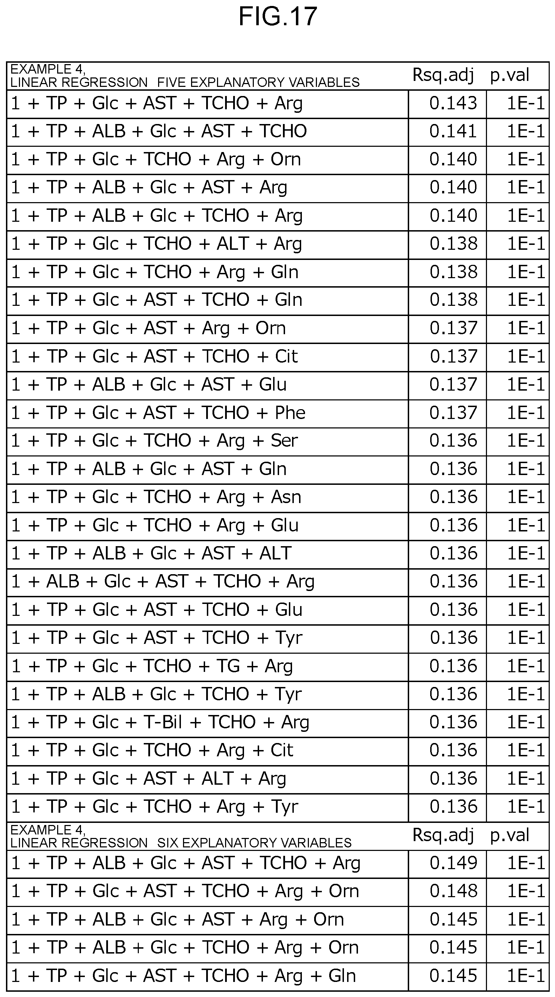

First, linear regression models were prepared. Prediction formulae with 1 to 6 explanatory variables to predict the haptoglobin concentration 7 days after the date of parturition were prepared using the measurement values 21 days before the expected date of parturition (the blood concentrations of the 25 kinds of amino acids and the concentration values of the 29 kinds of blood chemistry parameters). The prediction formulae prepared were evaluated in terms of R2 value and adjusted R2 value. In the case of one explanatory variable, the one explanatory variable was selected if the p-value was <0.05. In the case of two to six explanatory variables, the top 100 linear regression models in terms of adjusted R2 were extracted by extracting combinations of explanatory variables after narrowing down explanatory variables to those with p-values below a certain level for the one explanatory variable. FIG. 14 to FIG. 17 are tables of the linear regression models.

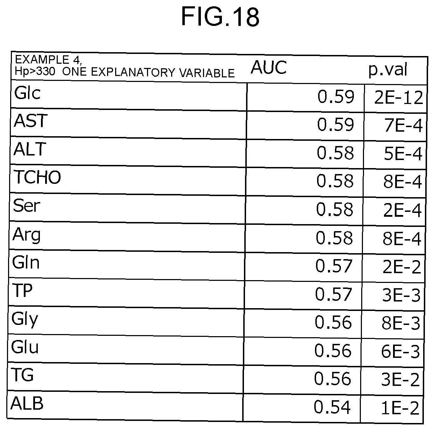

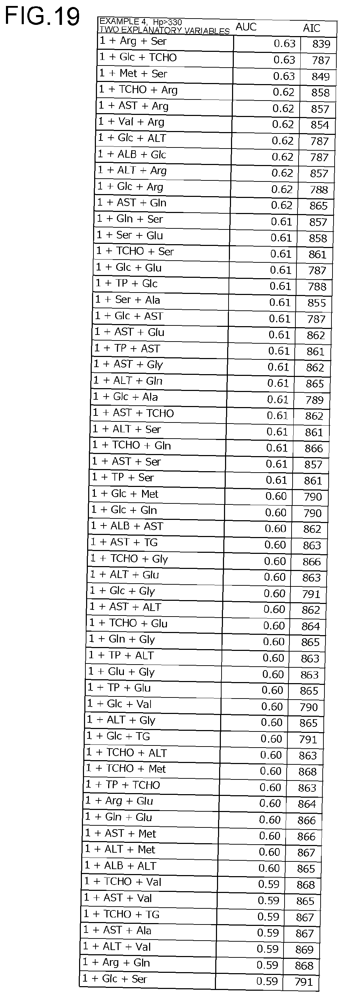

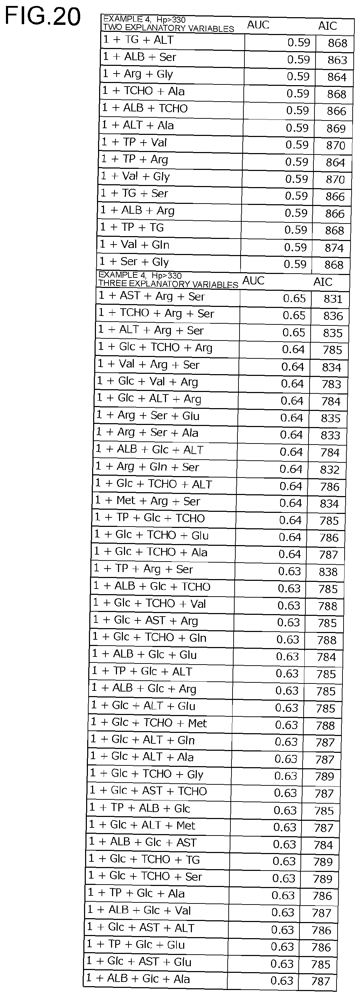

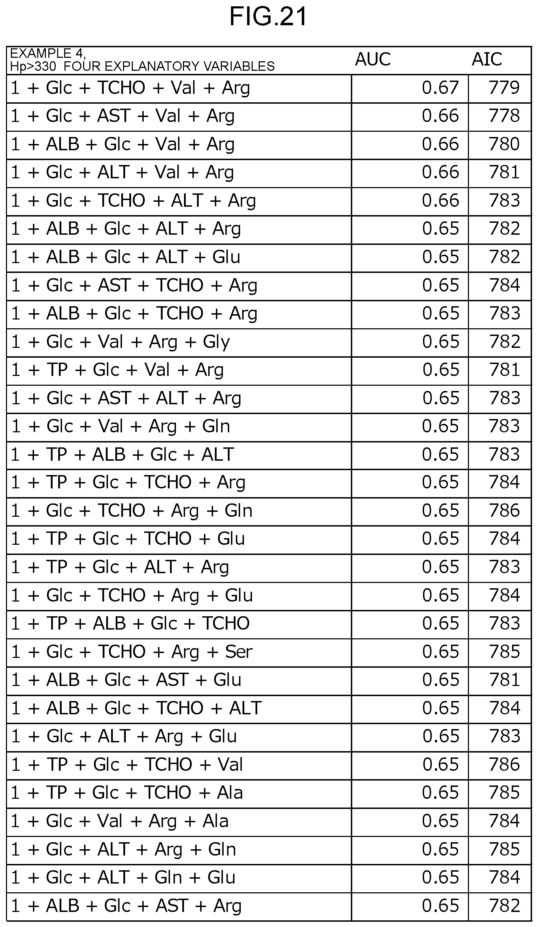

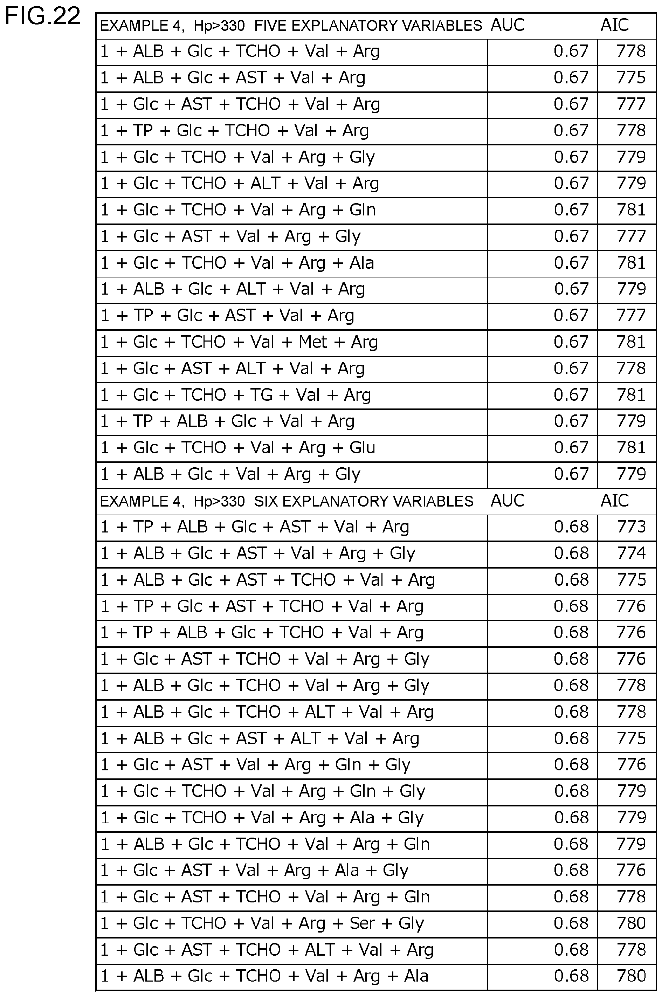

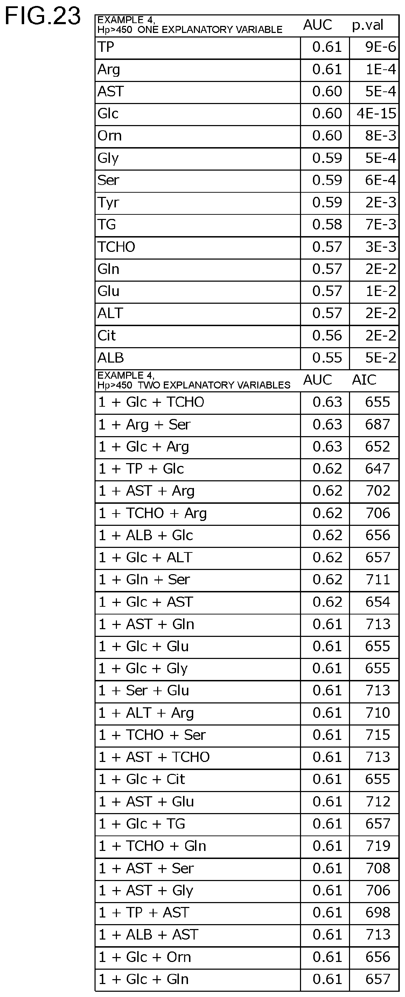

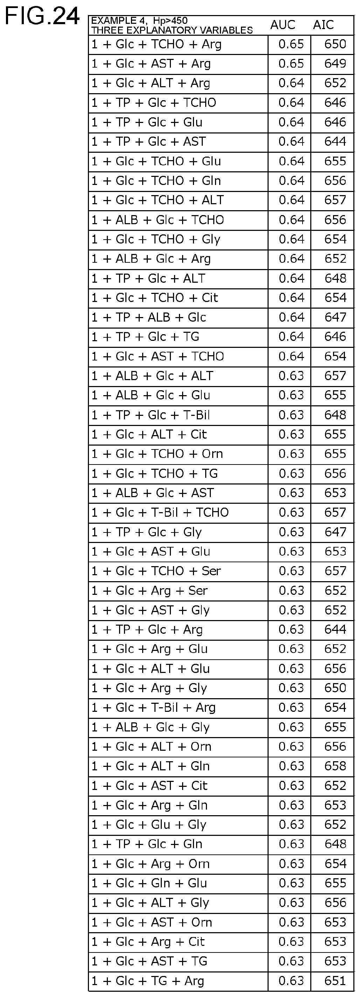

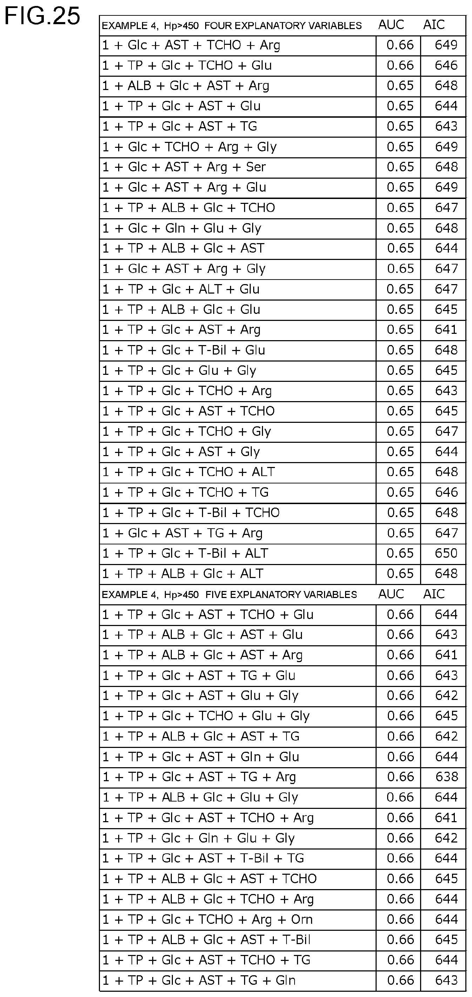

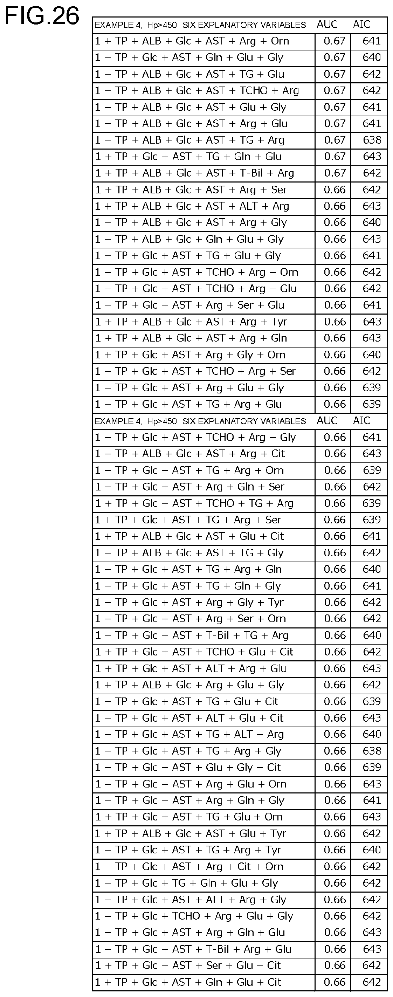

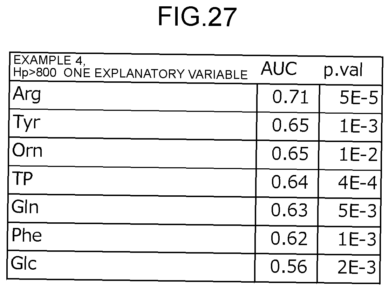

Next, logistic regression models were prepared. Three criteria (330, 450, 800 µg/ml) were set for the haptoglobin concentration 7 days after the date of parturition. Individuals with the haptoglobin concentration lower than each of the criterion values were classified into a healthy group, and individuals with the haptoglobin concentration equal to or higher than each of the criterion values were classified into a hyperinflammatory group. Then, an index with one to six explanatory variables that maximizes the discrimination ability for discriminating between the two groups was searched for by the logistic analysis (explanatory variable coverage method according to the minimum AIC) using the measurement values 21 days before the expected date of parturition, and the searched formulae were evaluated using the AUC of the ROC curve. In the case of one explanatory variable, the one explanatory variable was selected if the p-value was <0.05. In the case of two to six explanatory variables, the top 100 logistic regression models in terms of AIC were extracted by extracting combinations of explanatory variables after narrowing down explanatory variables to those with p-values below a certain level for the one explanatory variable. FIG. 18 to FIG. 27 are tables of the logistic regression models.

### Example 5

At 19 dairy farms, a total of 1428 blood samples were collected from a total of 714 female Holstein dairy cows 21 days before the expected date of parturition and 7 days after the date of parturition. The 714 dairy cows include 99 first-calf heifers (nulliparous cows) having no experience of parturition other than the present parturition and 615 parous cows having an experience of parturition in addition to the present parturition.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of the 29 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), Glob (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood samples collected 7 days after the date of parturition were measured to determine the concentration value of Ca (blood concentration (mg/dl)). The blood concentrations of the amino acids were measured by the measuring method (B).

Of the dataset for the 714 cows, those with missing values, component concentrations outside a quantitative range, or deaths during the study were excluded.

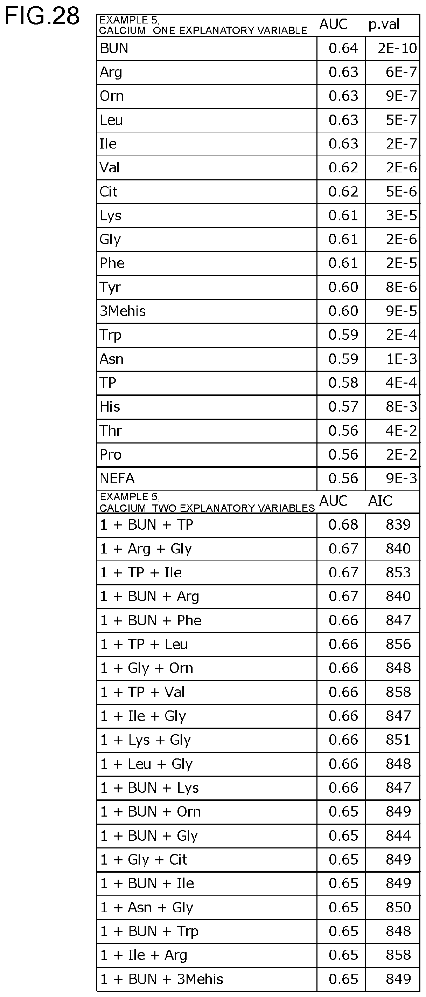

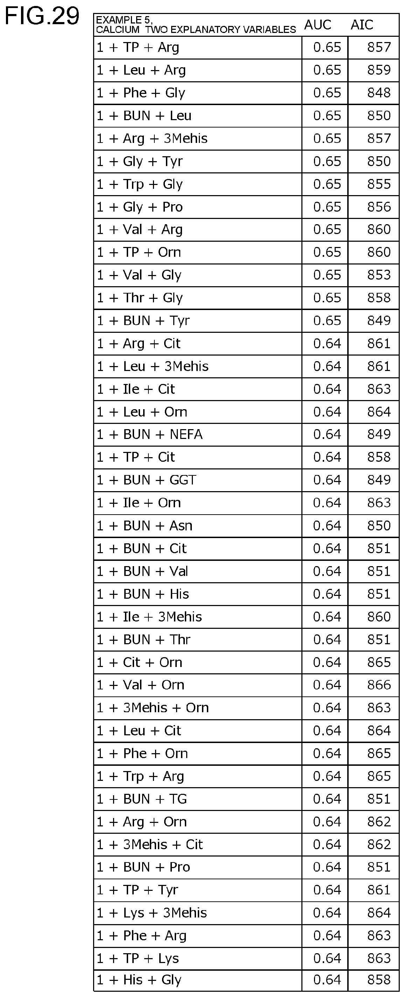

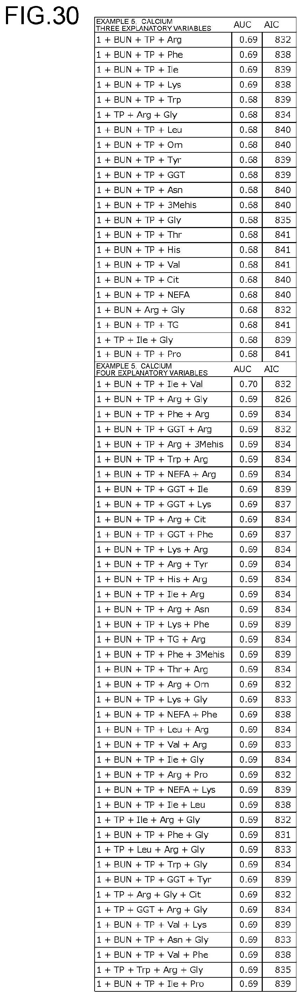

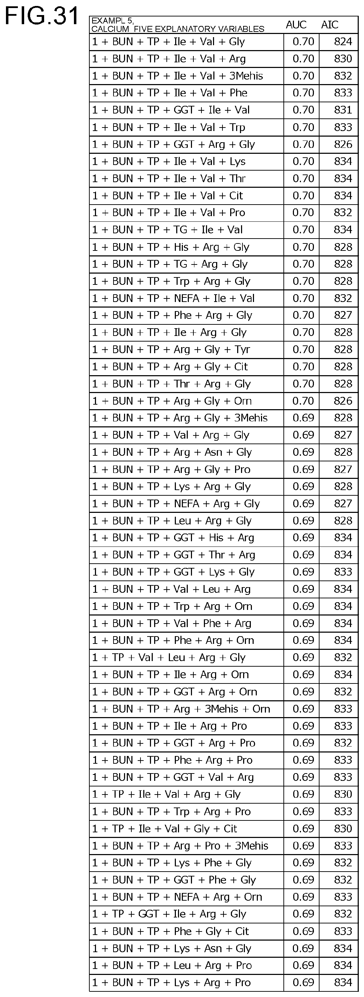

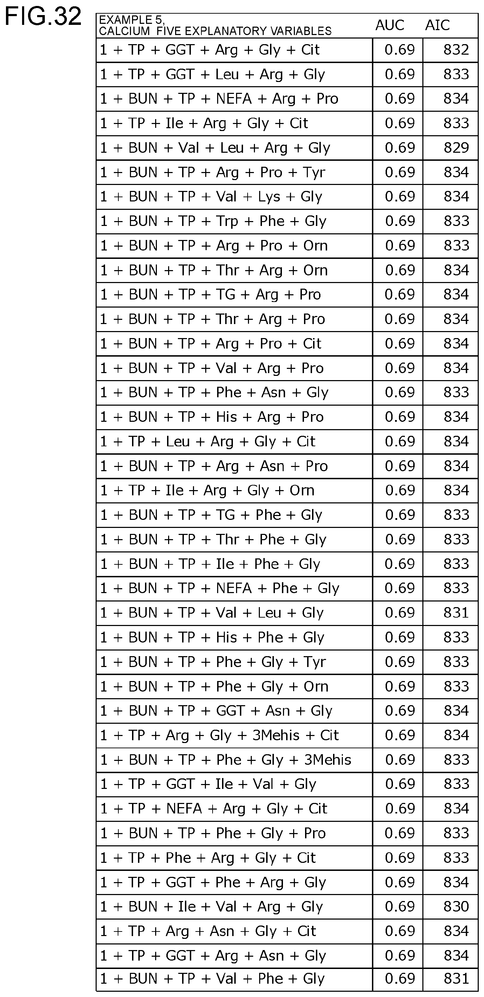

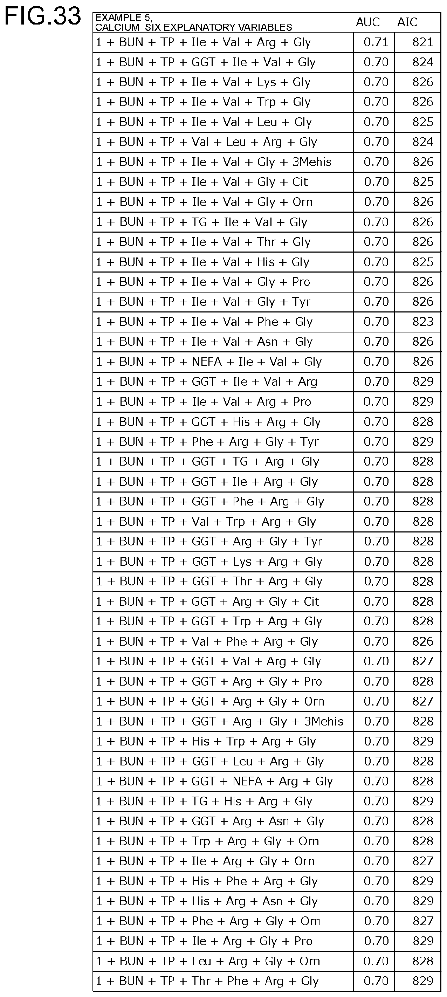

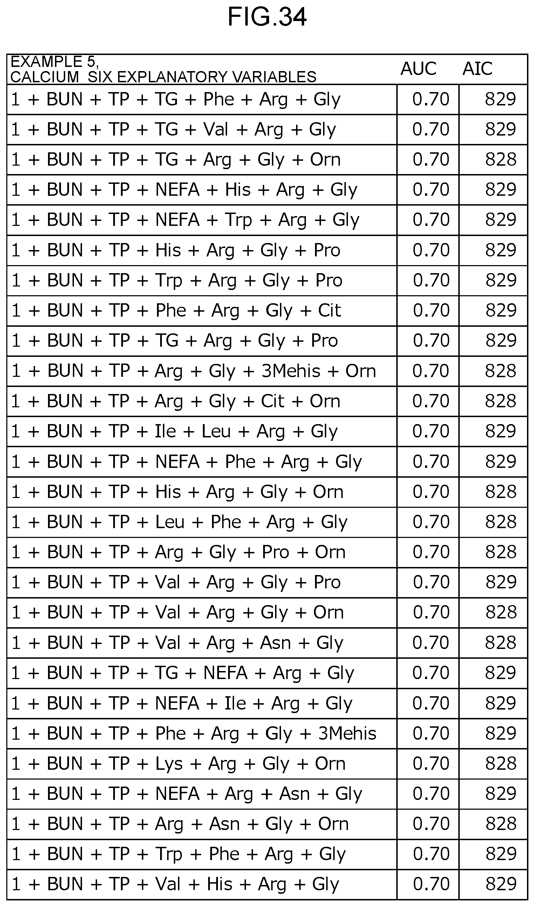

Logistic regression models were prepared. A criterion of 7.4 mg/dl was provided for the Ca concentration 7 days after the date of parturition. Individuals with the Ca concentration higher than the criterion value were classified into a healthy group, and individuals with the Ca concentration equal to or lower than the criterion value were classified into a low-Ca group. Then, an index with one to six explanatory variables that maximizes the discrimination ability for discriminating between the two groups was searched for by the logistic analysis (explanatory variable coverage method according to the minimum AIC) using the measurement values 21 days before the expected date of parturition, and the searched formulae were evaluated using the AUC of the ROC curve. In the case of one explanatory variable, the one explanatory variable was selected if the p-value was <0.05. In the case of two to six explanatory variables, the top 100 logistic regression models in terms of AIC were extracted by extracting combinations of explanatory variables after narrowing down explanatory variables to those with p-values below a certain level for the one explanatory variable. FIG. 28 to FIG. 34 are tables of the logistic regression models.

### Example 6

At one dairy farm, a total of 1038 blood samples were collected from a total of 519 female Holstein dairy cows 21 days before the expected date of parturition and 7 days after the date of parturition. The 519 dairy cows include 210 first-calf heifers (nulliparous cows) having no experience of parturition other than the present parturition and 309 parous cows having an experience of parturition in addition to the present parturition.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val) and the concentration values of the 29 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), Glob (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE). The blood concentrations of the amino acids were measured by the measuring method (A) or (B).

Of the dataset for the 519 cows, those with missing values, component concentrations outside a quantitative range, or deaths during the study were excluded.

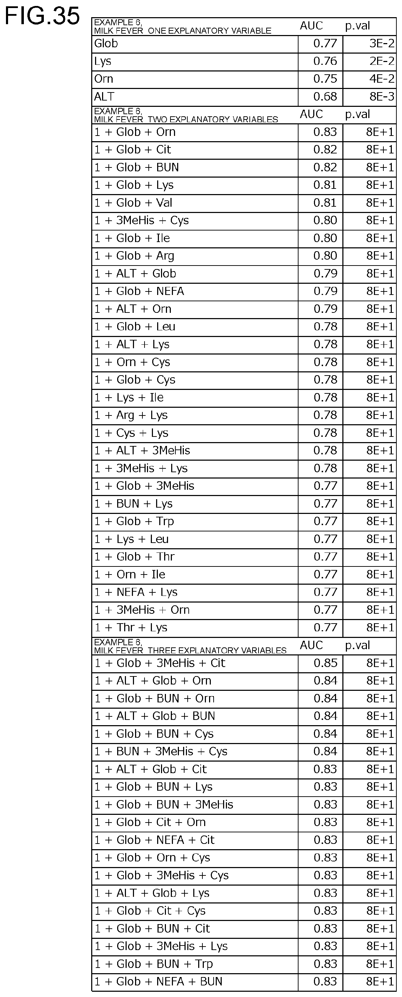

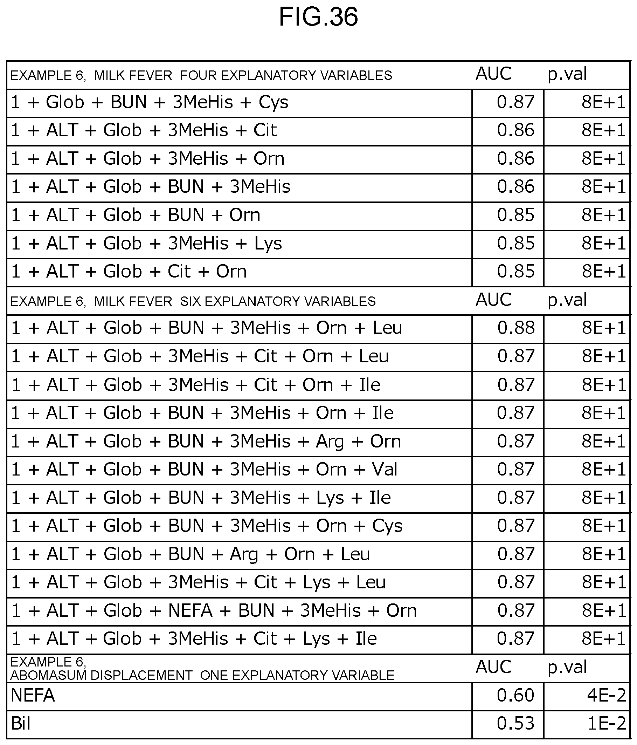

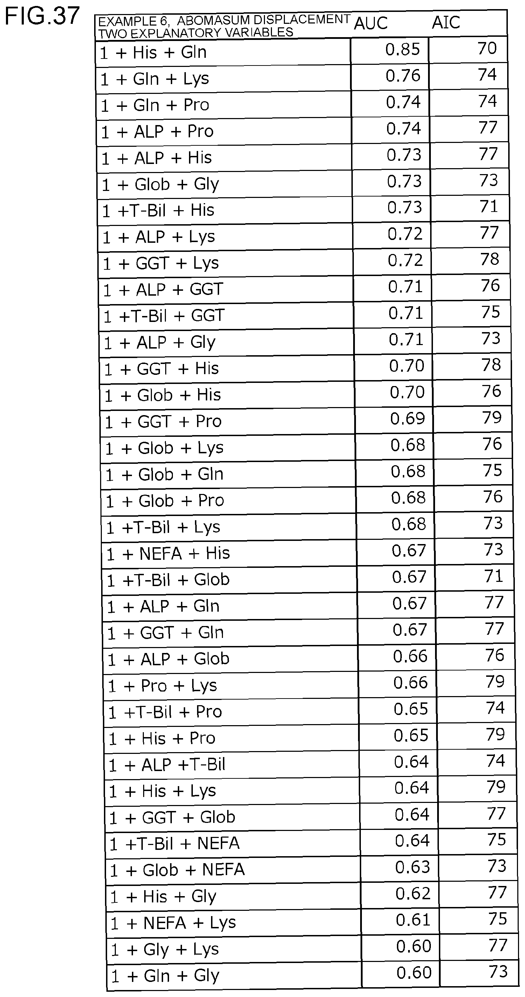

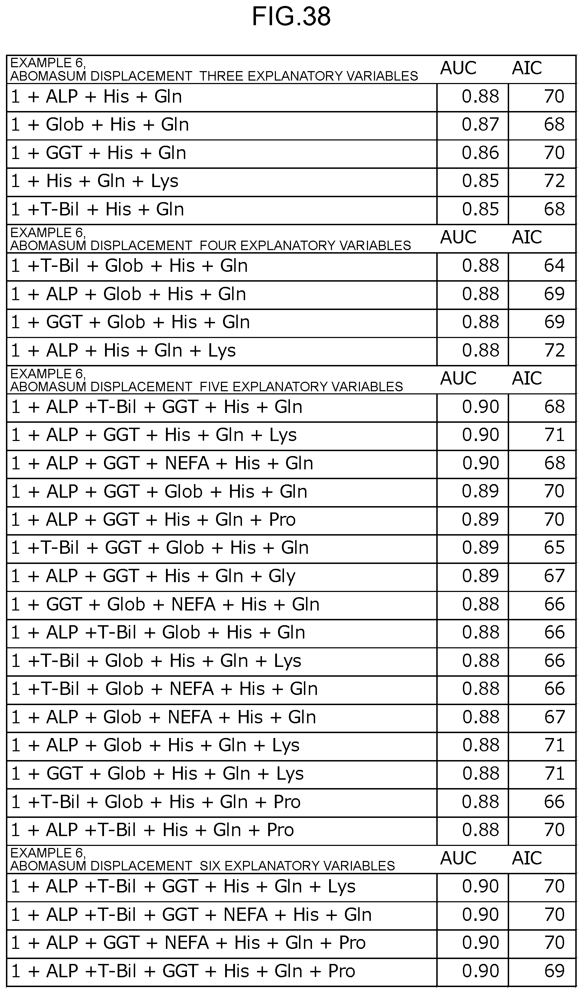

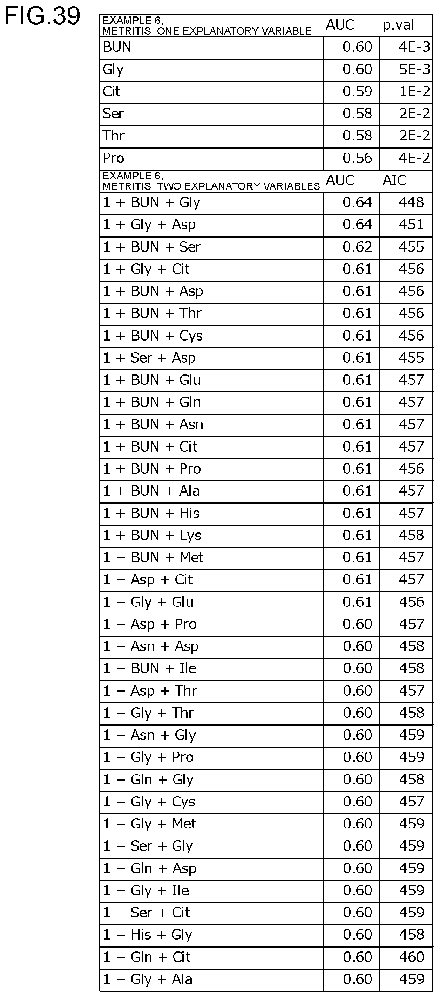

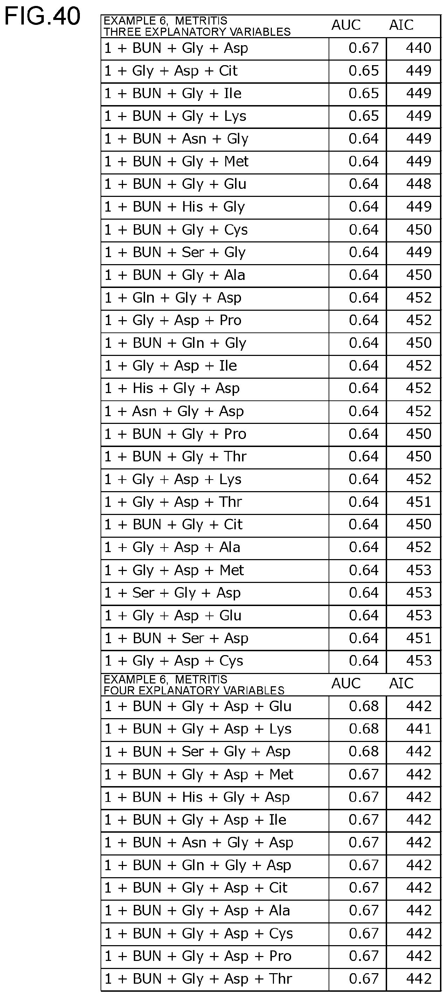

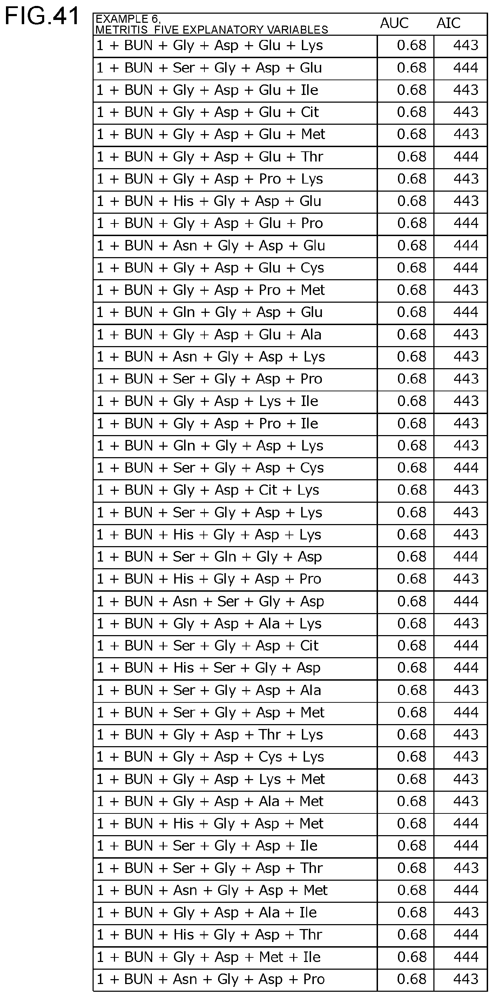

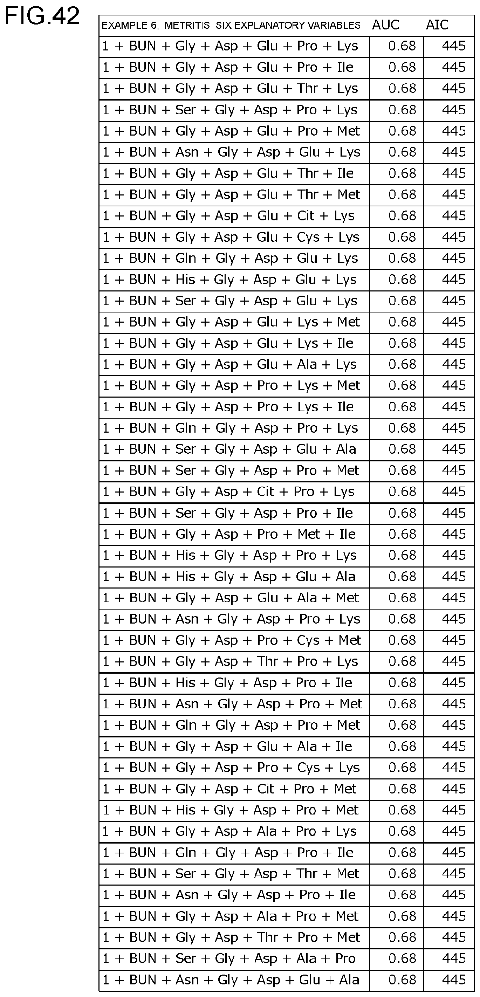

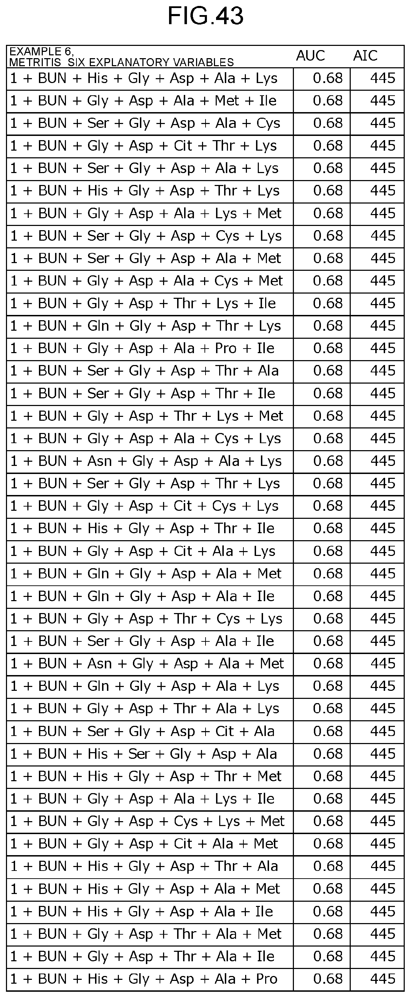

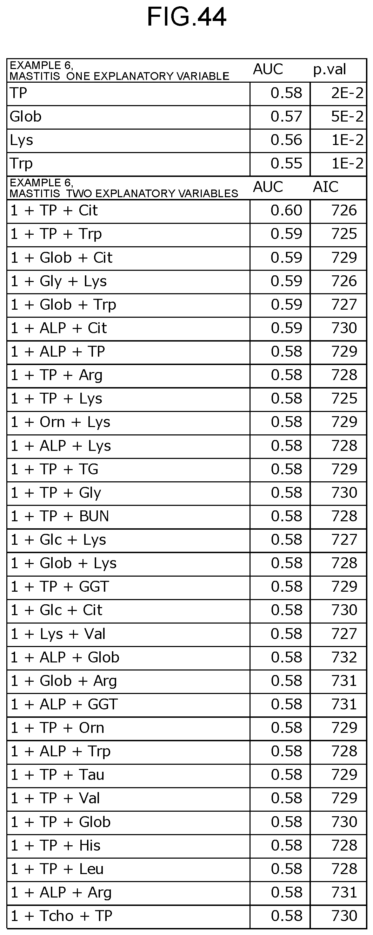

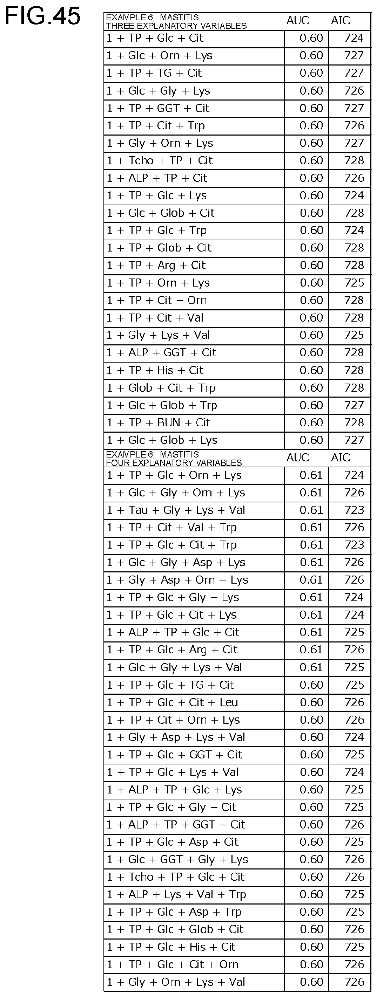

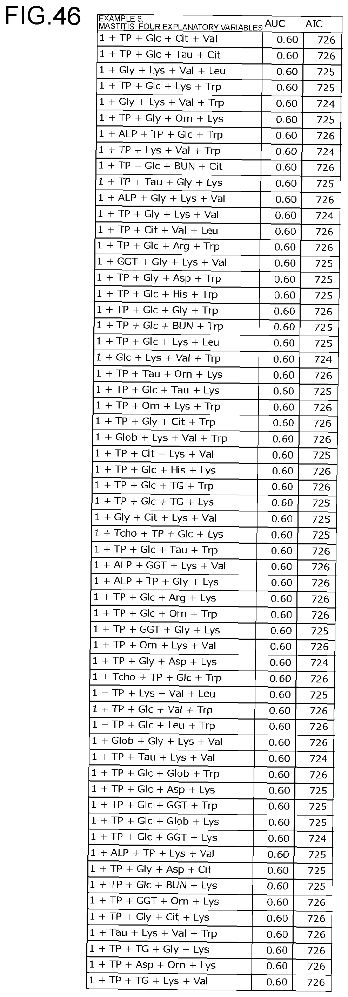

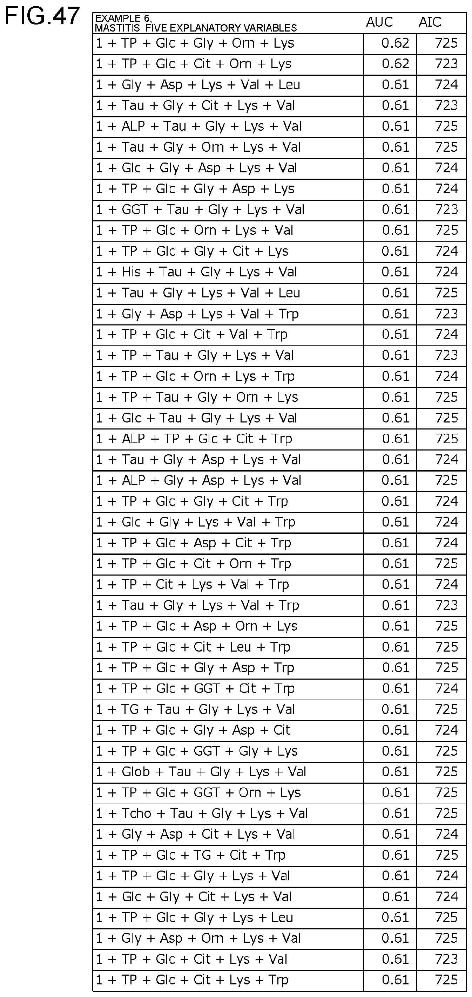

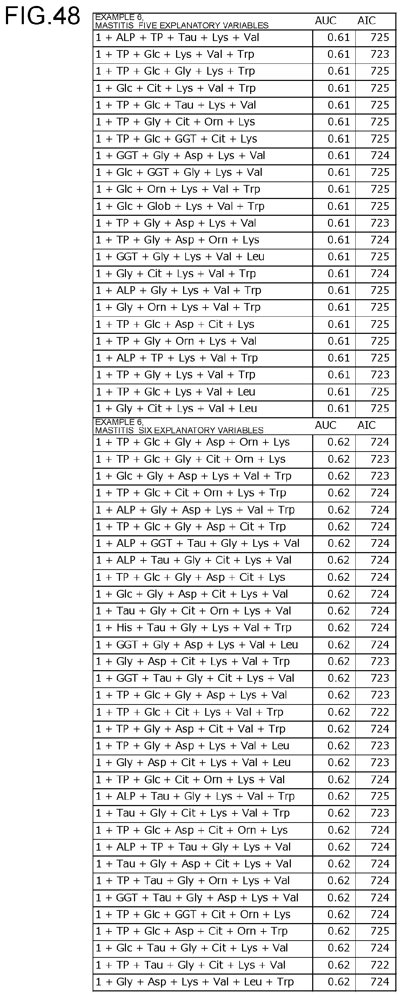

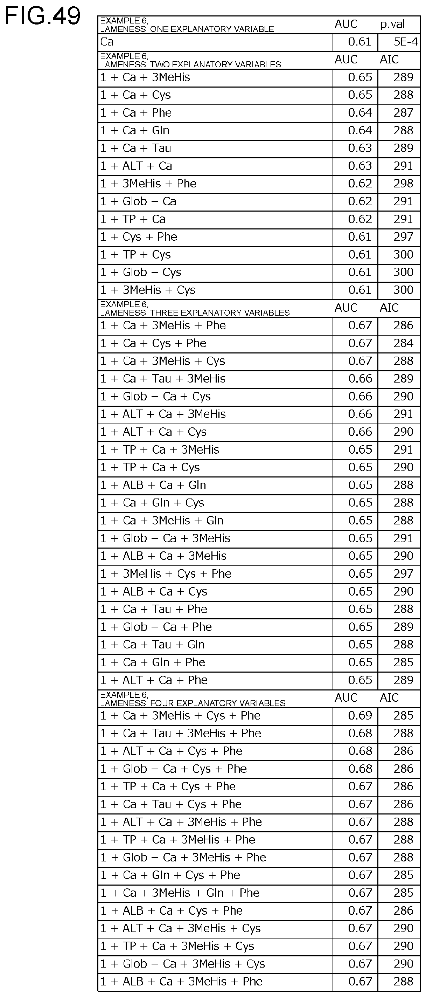

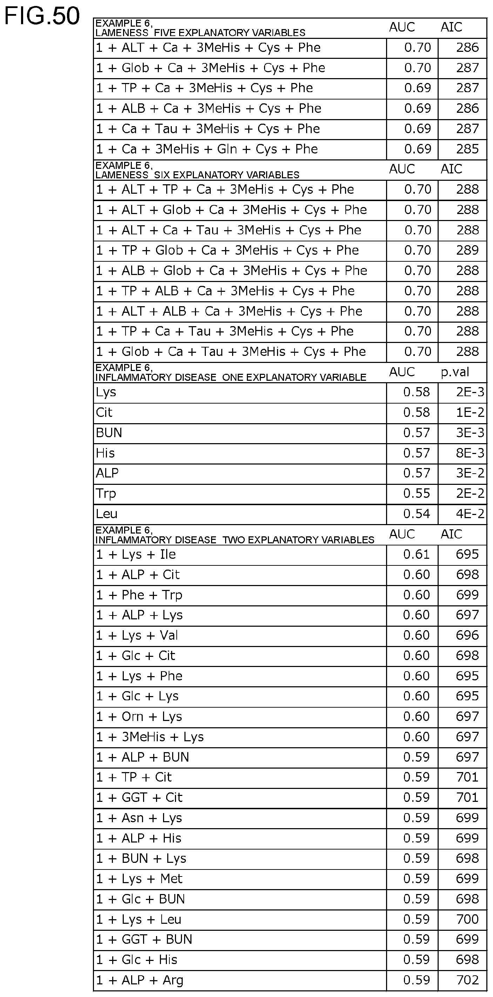

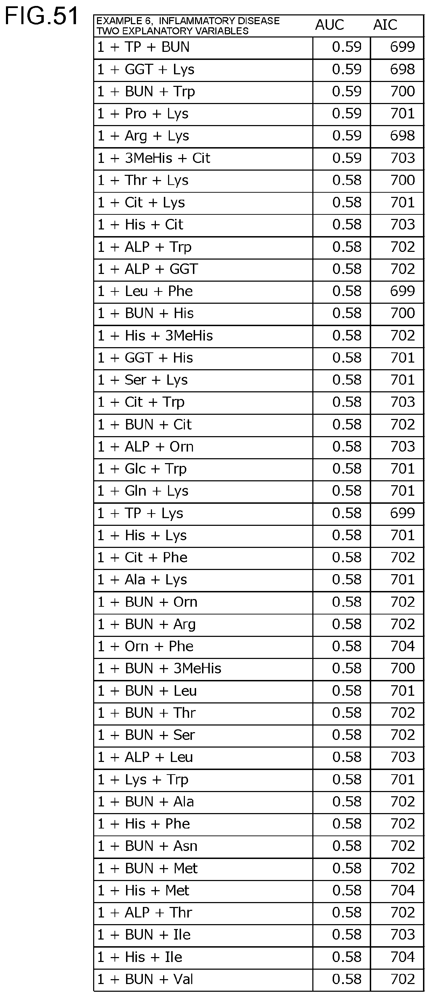

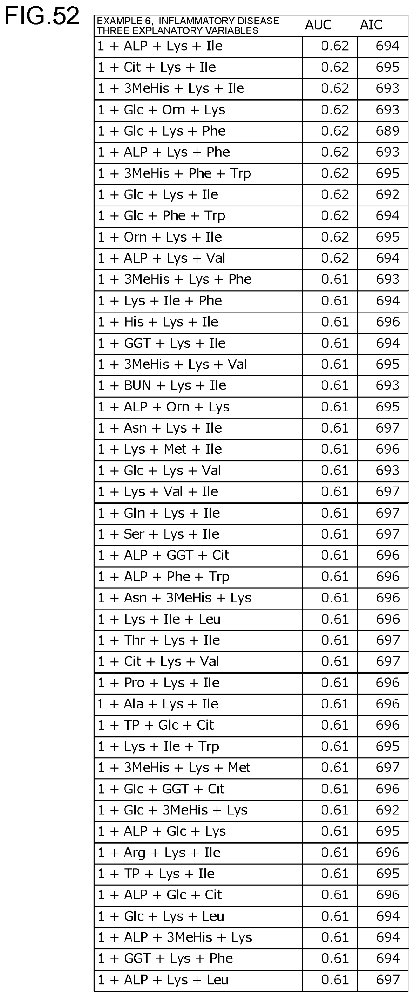

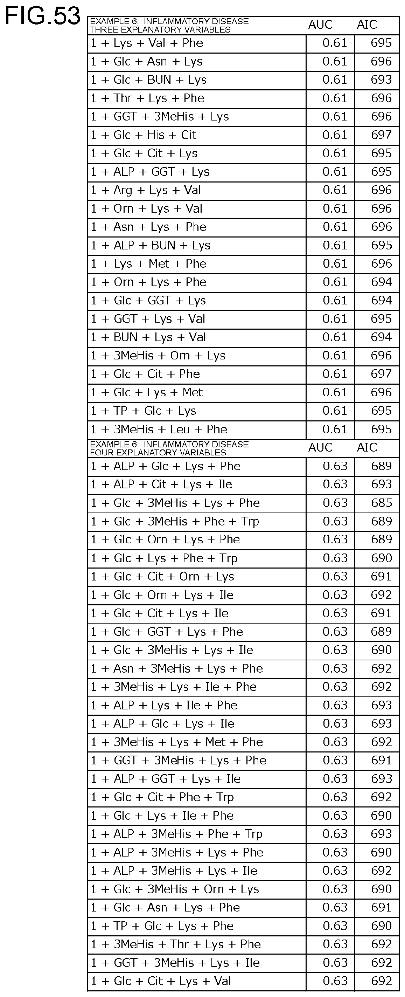

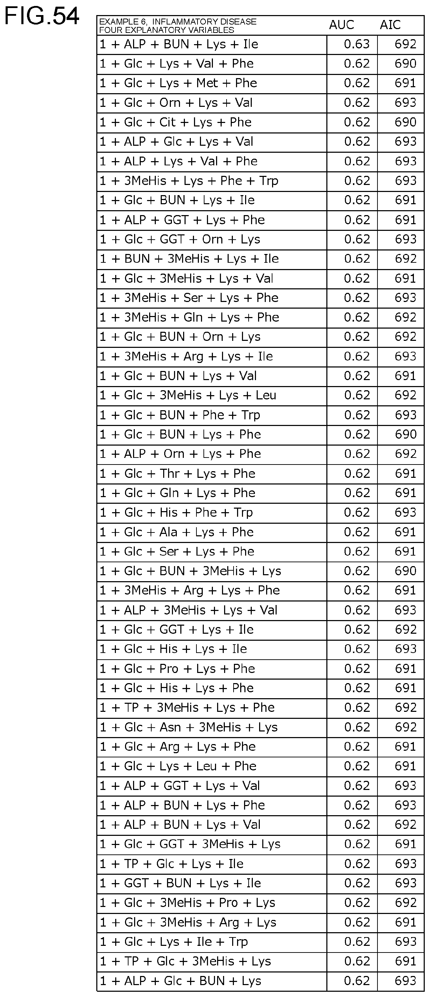

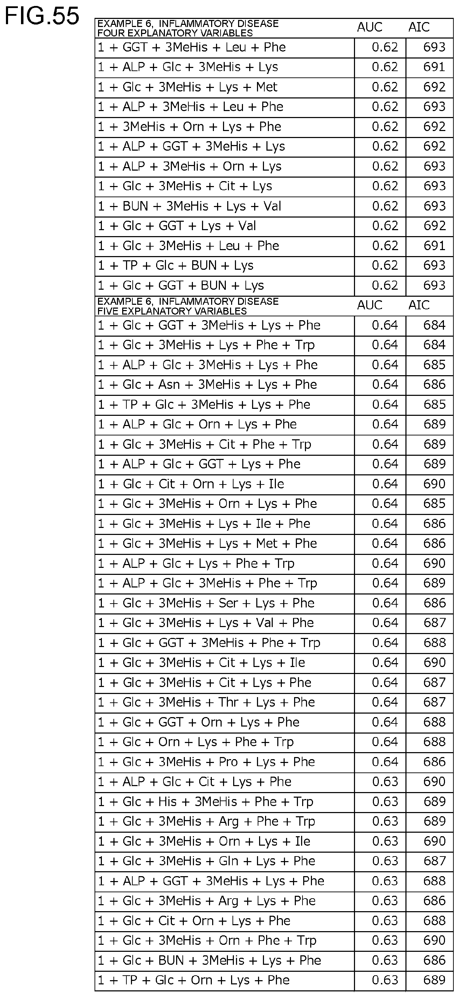

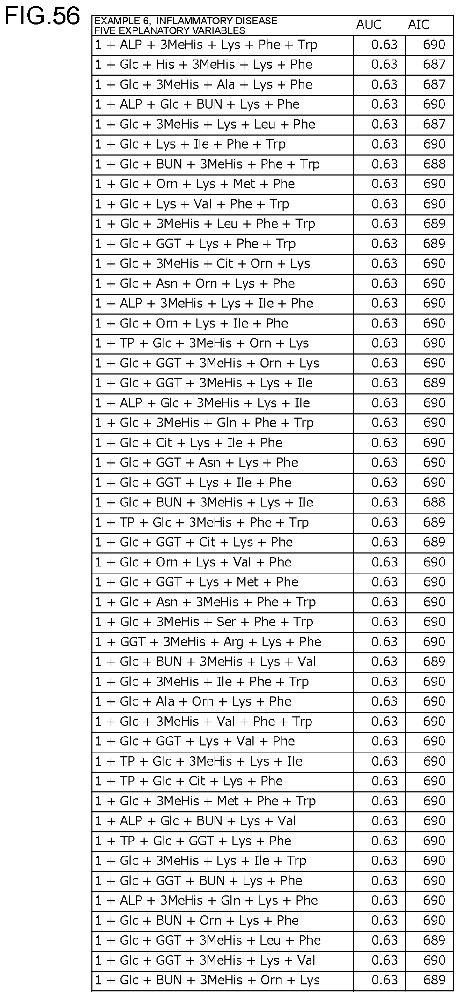

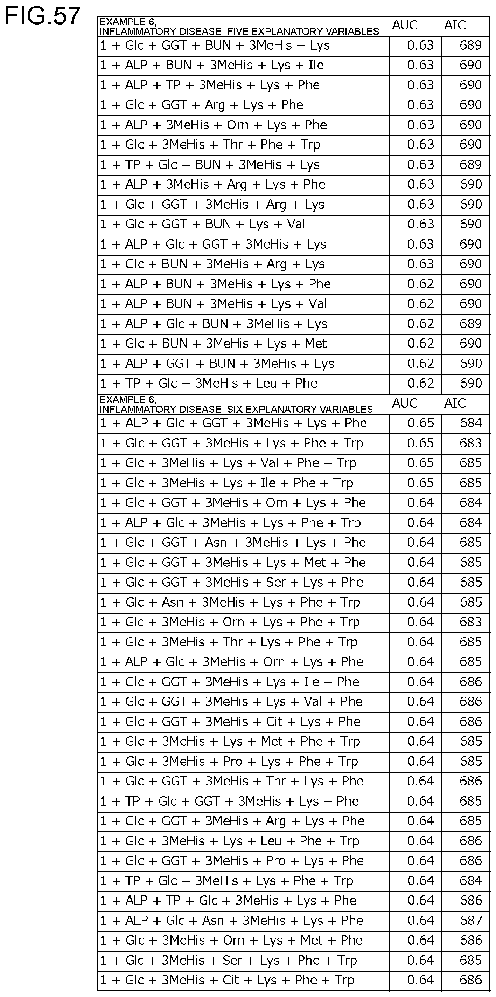

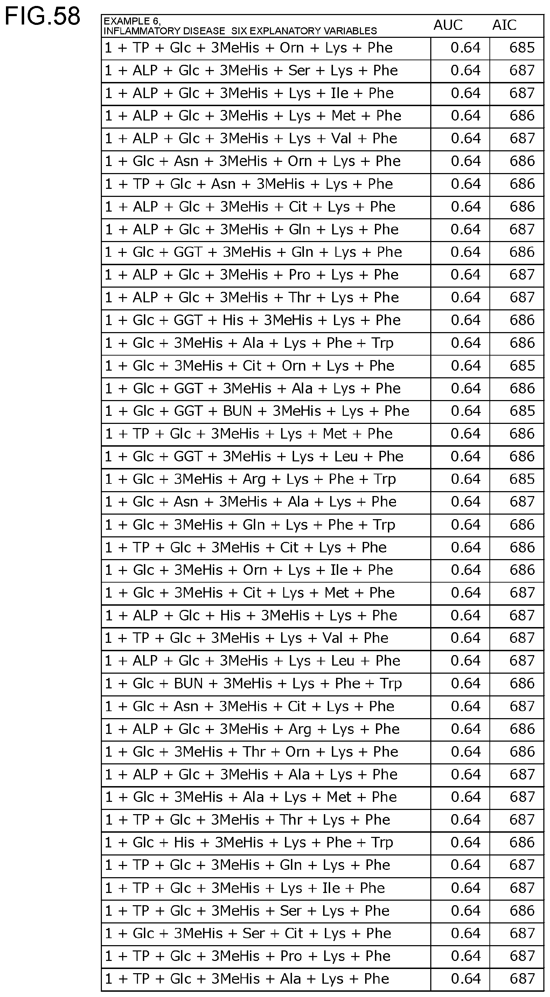

Logistic regression model equations were prepared. The individuals were classified into a healthy group and a diseased group according to the presence or absence of each disease of milk fever, abomasum displacement, metritis, mastitis, and lameness after parturition. Then, an index with one to six explanatory variables that maximizes the discrimination ability for discriminating between the two groups was searched for by the logistic analysis (explanatory variable coverage method according to the minimum AIC) using the measurement values 21 days before the expected date of parturition, and the searched formulae were evaluated using the AUC of the ROC curve. In the case of one explanatory variable, the one explanatory variable was selected if the p-value was <0.05. In the case of two to six explanatory variables, the top 100 logistic regression models in terms of AIC were extracted by extracting combinations of explanatory variables after narrowing down explanatory variables to those with p-values below a certain level for the one explanatory variable. FIG. 35 and FIG. 36 are tables of the logistic regression models for milk fever discrimination. FIG. 36 to FIG. 38 are tables of the logistic regression models for abomasum displacement. FIG. 39 to FIG. 43 are tables of the logistic regression models for metritis discrimination. FIG. 44 to FIG. 48 are tables of the logistic regression models for mastitis discrimination. FIG. 49 and FIG. 50 are tables of the logistic regression models for lameness discrimination. FIG. 50 to FIG. 59 are tables of the logistic regression models for discriminating postpartum development of any inflammatory disease among milk fever, abomasum displacement, metritis, mastitis, lameness, and ketosis.

### Example 7

At one dairy farm, a total of 80 blood samples were collected from a total of 40 female Holstein dairy cows 21 days before the expected date of parturition and 7 days after the date of parturition. All the 40 cows are parous cows having an experience of parturition in addition to the present parturition.

The blood samples collected 21 days before the expected date of parturition were measured to determine the blood concentrations of the 25 kinds of amino acids (Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, Val), the concentration values of the 29 kinds of blood chemistry parameters (ALB (g/dl), ALT (IU/l), AST (IU/l), BHBA (µmol/l), BUN (mg/dl), Ca (mg/dl), gGTP (IU/l), Glc (mg/dl), Glob (mg/dl), NEFA (µEq/l), T-Bil (mg/dl), TCHO (mg/dl), TG (mg/dl), TP (g/dl), P, Na, K, Cl, GGT, ALP, LDH, CPK, LDL-C, HDL-C, Mg, AMY, PA, LA, CRE), and the measurement values of two kinds of measurement items (the d-ROMs value, the BAP value). The blood samples collected 7 days after the date of parturition were measured to determine the blood concentrations of serum amyloid A (SAA) and IL-1β as well as the d-ROMs and BAP values. The blood concentrations of the amino acids were measured by the measuring method (A) or (B). The blood concentrations of serum amyloid A and IL-1β were measured by a measuring method called enzyme-linked immuno sorbent assay (ELISA). The d-ROMs and BAP values were measured by a measuring method called colorimetric method.

Of the dataset for the 40 cows, those with missing values, component concentrations outside a quantitative range, or deaths during the study were excluded.

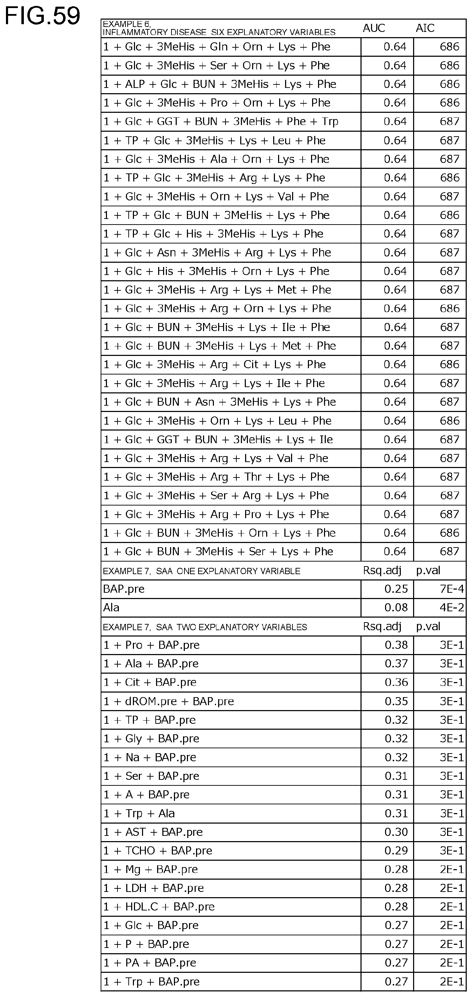

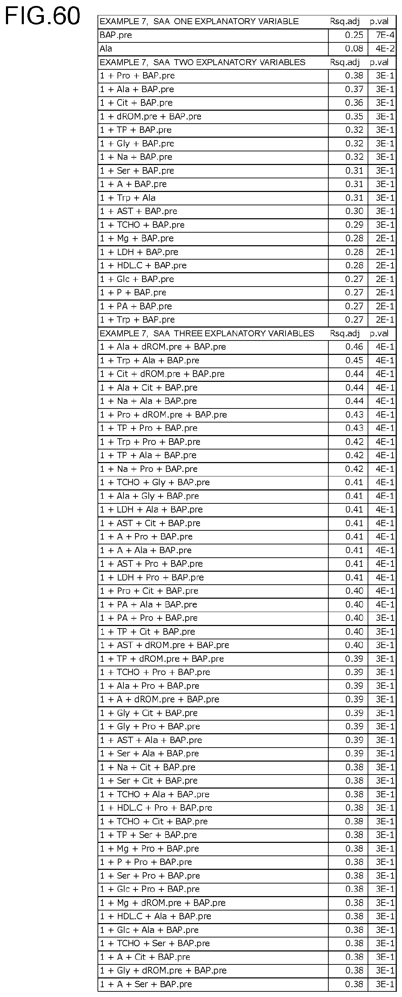

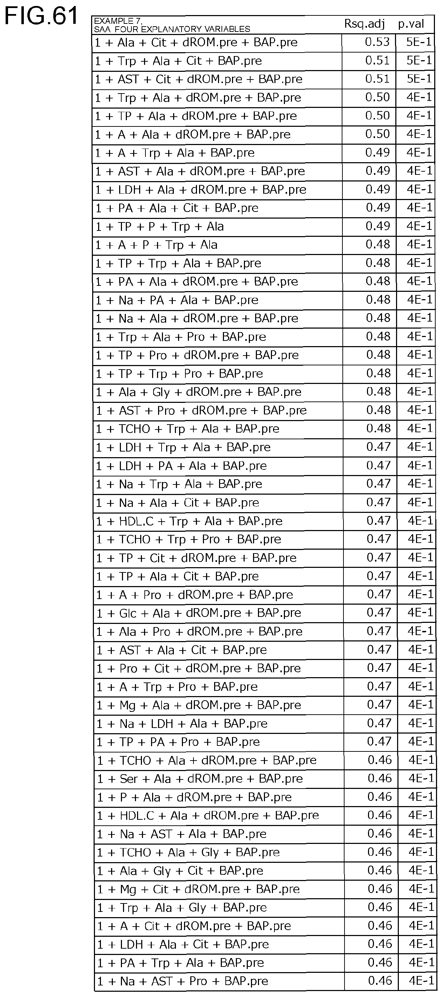

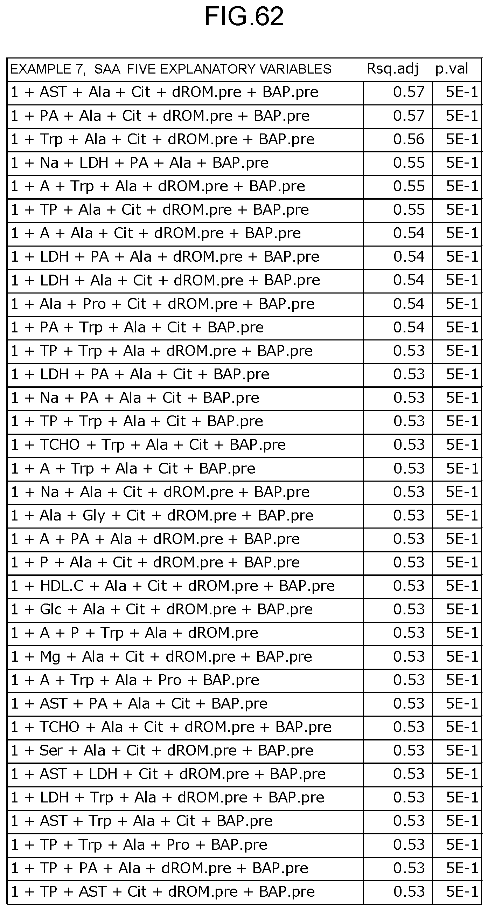

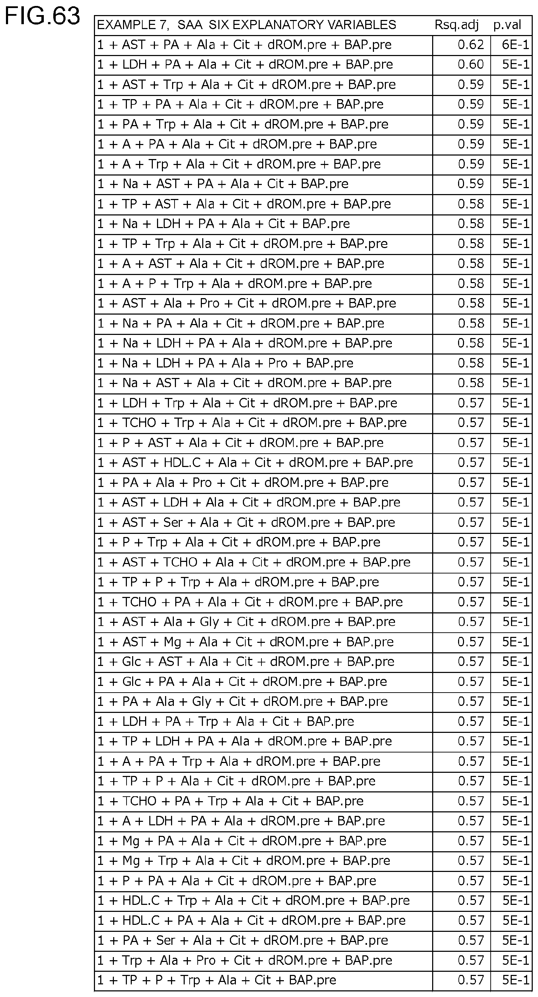

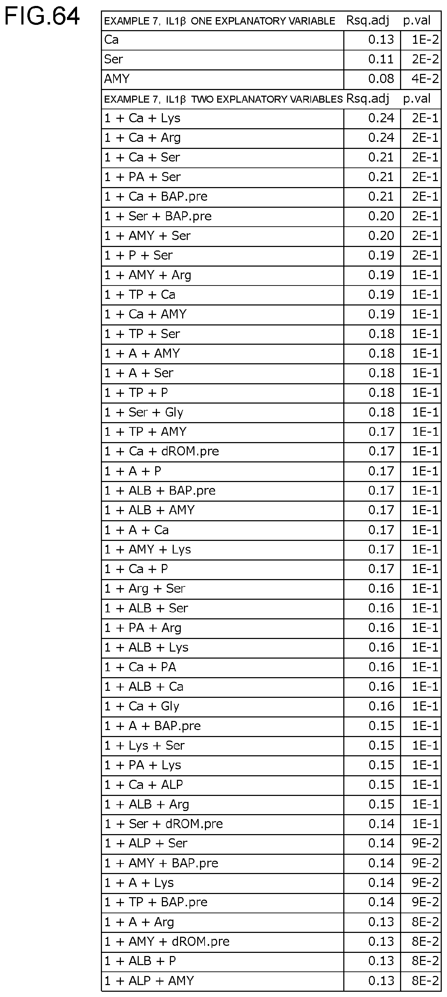

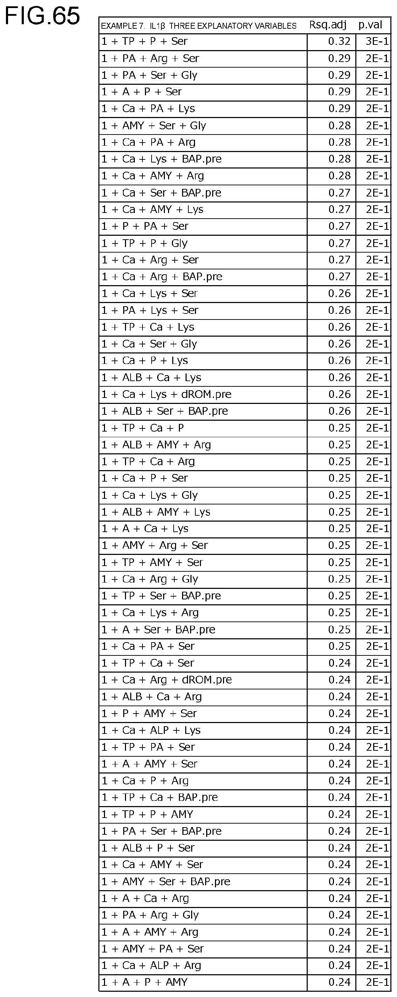

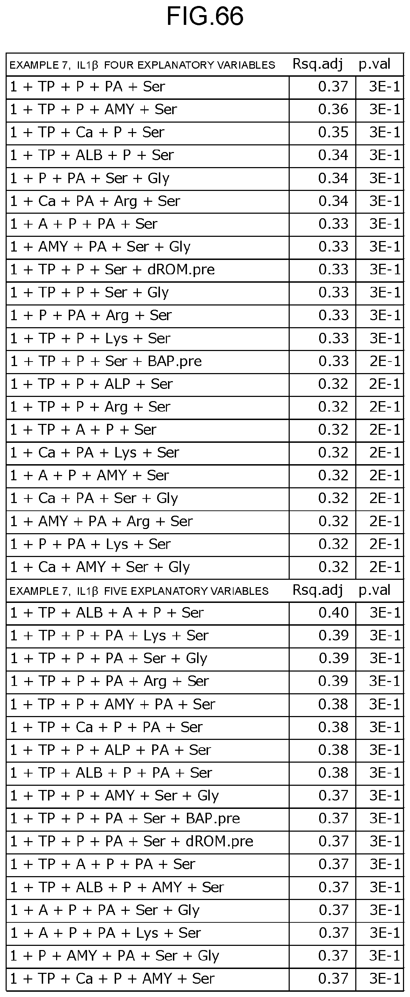

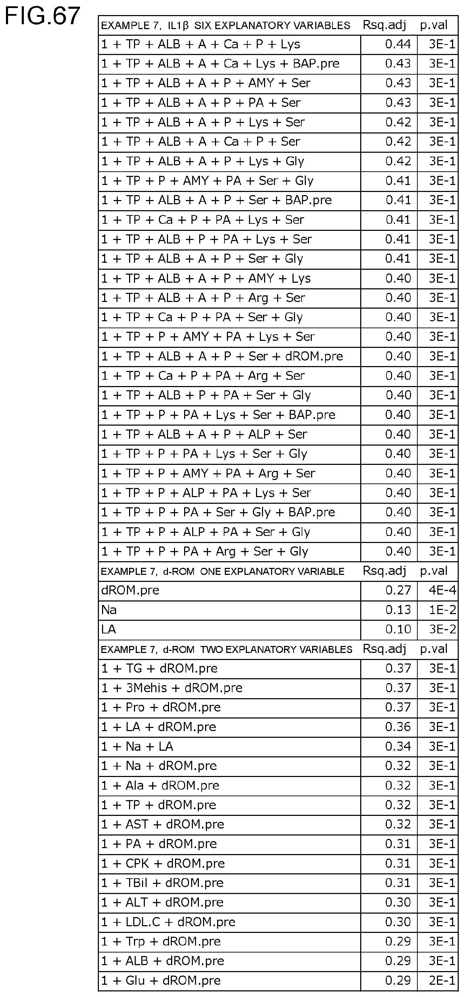

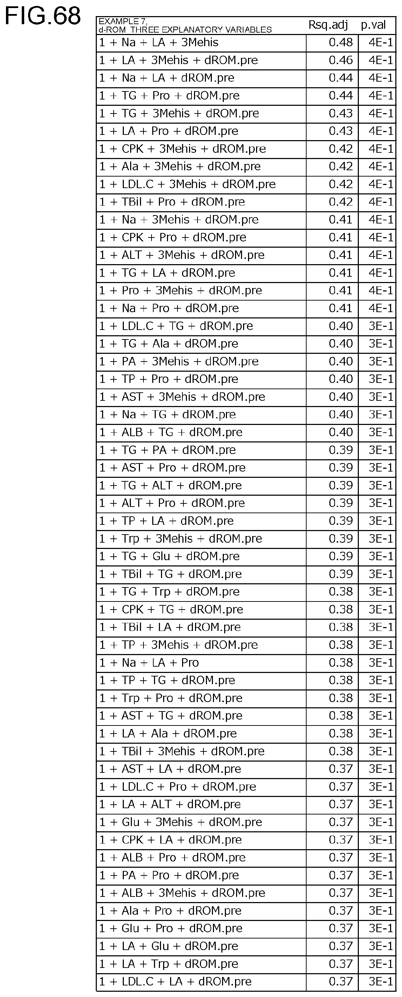

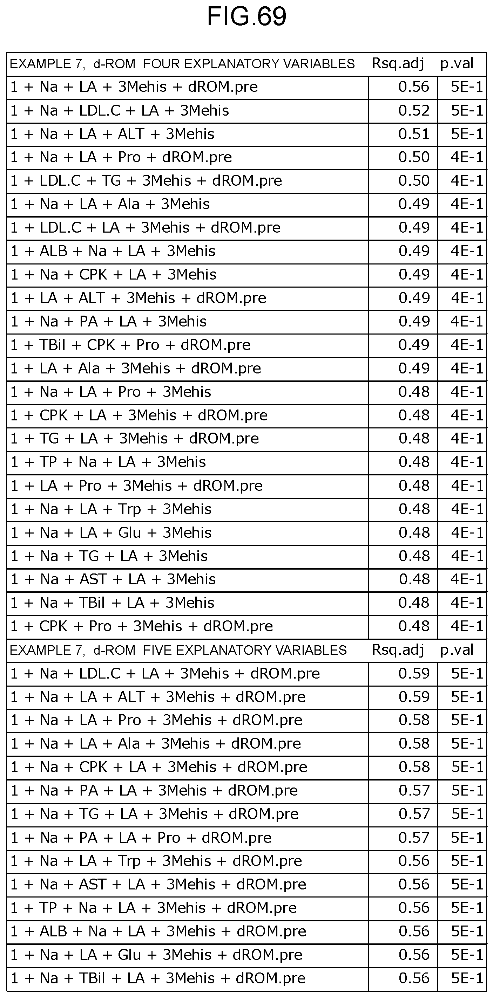

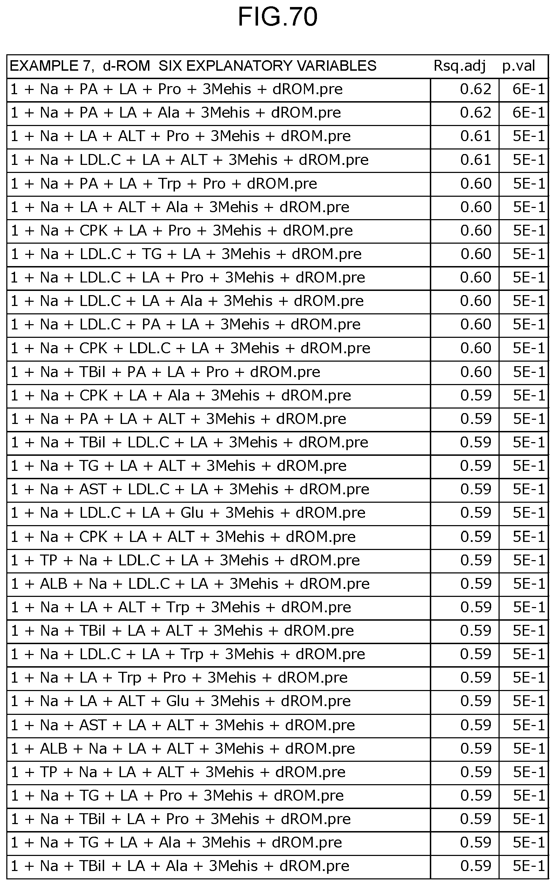

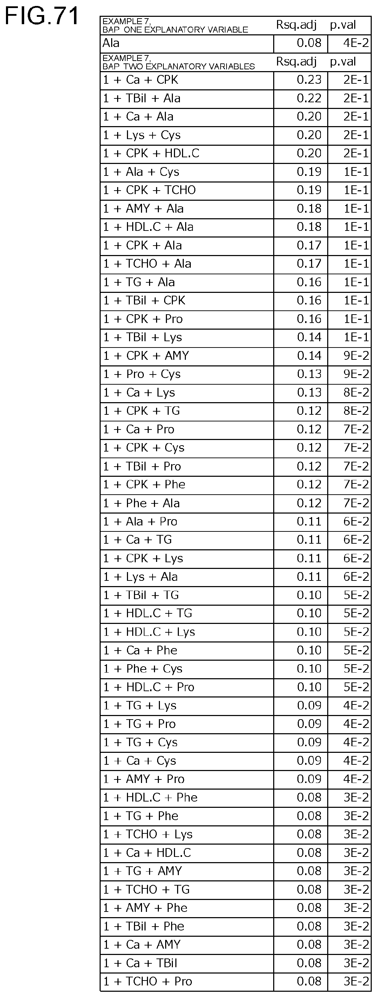

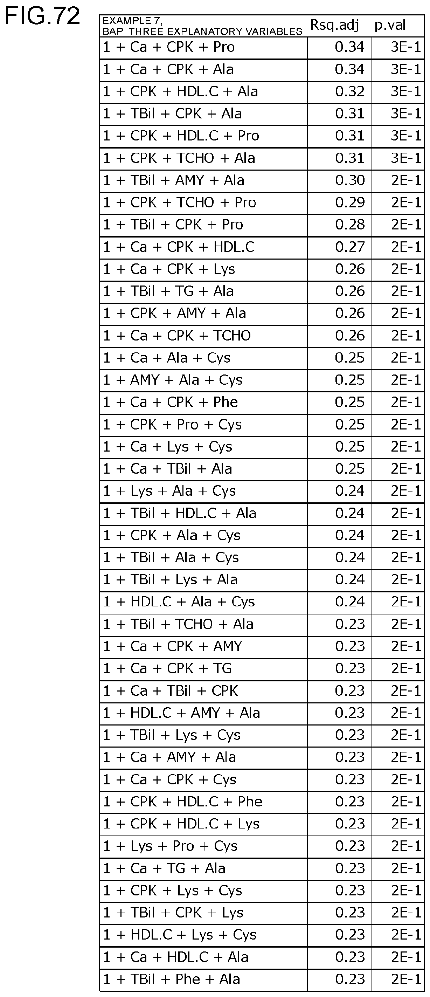

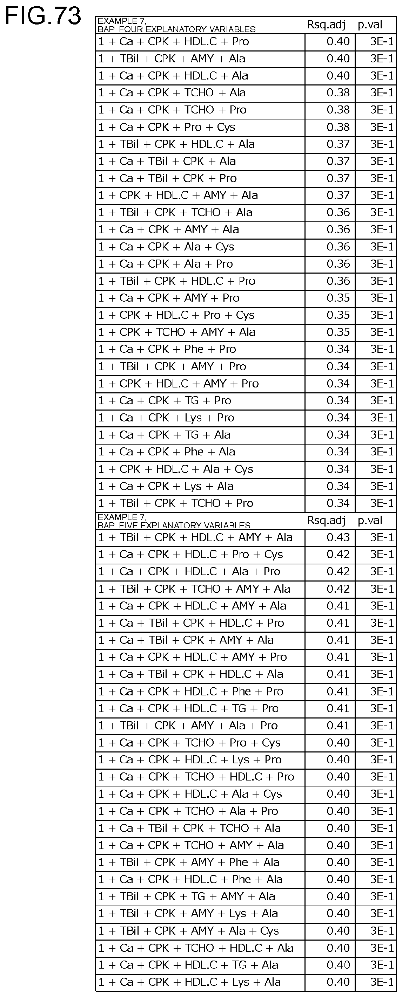

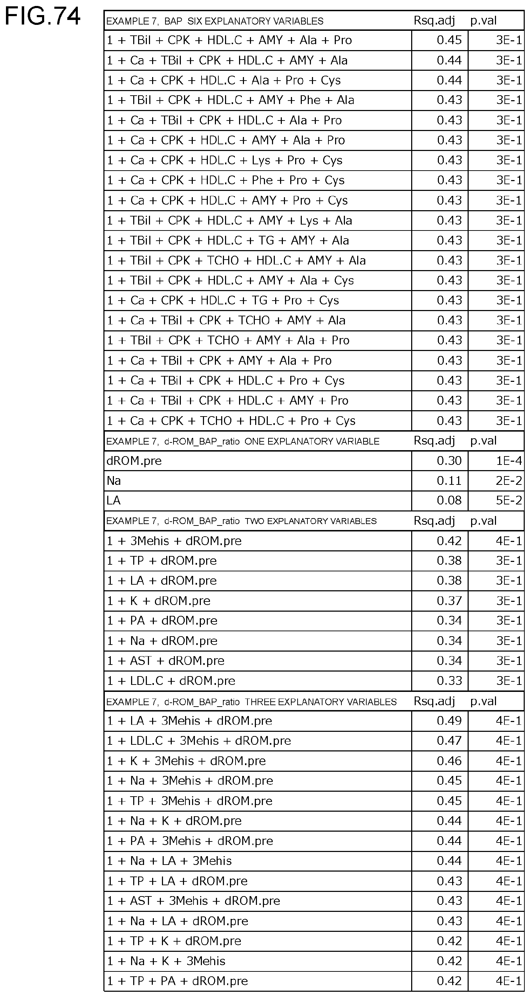

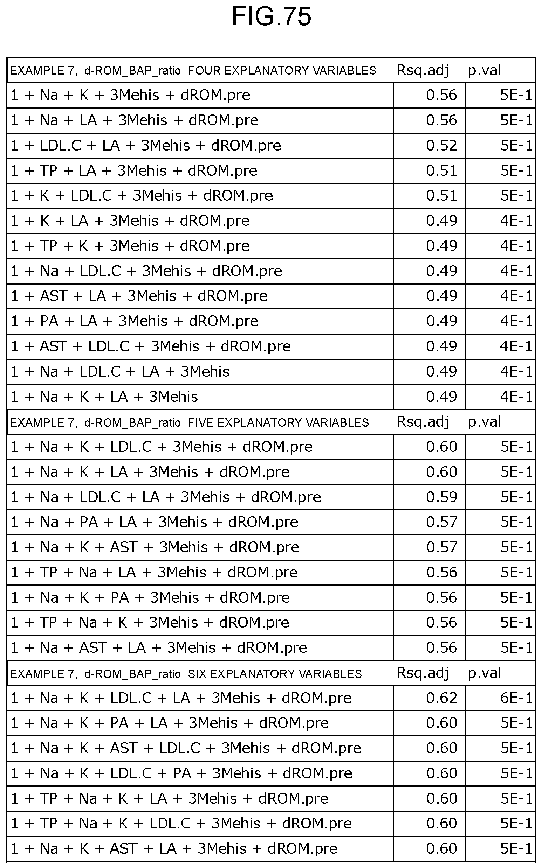

Linear regression models were prepared. Prediction formulae with one to six explanatory variables to predict the serum amyloid A concentration 7 days after the date of parturition, prediction formulae with one to six explanatory variables to predict the IL-1β concentration 7 days after the date of parturition, prediction formulae with one to six explanatory variables to predict the d-ROMs value 7 days after the date of parturition, prediction formulae with one to six explanatory variables to predict the BAP value 7 days after the date of parturition, and prediction formulae with one to six explanatory variables to predict the d-ROMs value/BAP value 7 days after the date of parturition were prepared using the measurement values 21 days before the expected date of parturition (the blood concentrations of the 25 kinds of amino acids, the concentration values of the 29 kinds of blood chemistry parameters, and the measurement values of the two kinds of measurement items). The prediction formulae prepared were evaluated in terms of adjusted R2 value. In the case of one explanatory variable, the one explanatory variable was selected if the p-value was <0.05. In the case of two to six explanatory variables, the top linear regression models in terms of adjusted R2 were extracted by extracting combinations of explanatory variables after narrowing down explanatory variables to those with p-values below a certain level for the one explanatory variable. FIG. 59 to FIG. 63 are tables of the linear regression models to predict the serum amyloid A concentration 7 days after the date of parturition. FIG. 64 to FIG. 67 are tables of the linear regression models to predict the IL-1β concentration 7 days after the date of parturition. FIG. 67 to FIG. 70 are tables of the linear regression models to predict the d-ROMs value 7 days after the date of parturition. FIG. 71 to FIG. 74 are tables of the linear regression models to predict the BAP value 7 days after the date of parturition. FIG. 74 and FIG. 75 are tables of the linear regression models to predict the d-ROMs value/BAP value 7 days after the date of parturition.

### INDUSTRIAL APPLICABILITY

As discussed above, the present invention is highly valuable in many industrial fields, in particular, dairy farming, development of medicine or feed for cattle, veterinary care for cattle, and the like.

### EXPLANATION OF LETTERS OR NUMERALS

- 100: Evaluating apparatus
- 102: Control device
- 102a: Obtaining part
- 102b: Designating part
- 102c: Formula-preparing part
- 102d: Evaluating part
- 102d1: Calculating part
- 102d2: Converting part
- 102d3: Generating part
- 102d4: Classifying part
- 102e: Result outputting part
- 102f: Sending part
- 104: Communication interface
- 106: Memory device
- 106a: Blood data file
- 106b: Index state information file
- 106c: Designated index state information file
- 106d: Formula-related information database
- 106d1: Formula file
- 106e: Evaluation result file
- 108: Input/output interface
- 112: Input device
- 114: Output device
- 200: Client apparatus (terminal apparatus (information communication terminal apparatus))
- 300: Network
- 400: Database apparatus

## Claims

1. An evaluating method comprising an evaluating step of evaluating a state of inflammation in a ruminant after parturition, using at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, or using a value of a formula calculated using the at least one value and the formula including an explanatory variable to be substituted with the at least one value.

2. The evaluating method according to claim 1, wherein the inflammation is metritis, mastitis, hypocalcemia, milk fever, abomasum displacement, or lameness.

3. The evaluating method according to claim 1 or 2, wherein the ruminant is a dairy cow or a beef cow.

4. The evaluating method according to claim 3, wherein the evaluating step is executed in a control unit of an information processing apparatus.

5. A calculating method comprising a calculating step of calculating a value of a formula for evaluating a state of inflammation in a ruminant after parturition, using (i) at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, and (ii) the formula including an explanatory variable to be substituted with the at least one value.

6. The calculating method according to claim 5, wherein the calculating step is executed in a control unit of an information processing apparatus.

7. An evaluating apparatus comprising an evaluating unit that evaluates a state of inflammation in a ruminant after parturition, using at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, or using a value of a formula calculated using the at least one value and the formula including an explanatory variable to be substituted with the at least one value.

8. The evaluating apparatus according to claim 7, wherein
the evaluating apparatus is communicably connected via a network to a terminal apparatus that provides the at least one value or the value of the formula,
the evaluating apparatus further comprises:
a data receiving unit that receives the at least one value or the value of the formula transmitted from the terminal apparatus; and
a result sending unit that transmits an evaluation result obtained by the evaluating unit to the terminal apparatus, and
the evaluating unit uses the at least one value or the value of the formula received by the data receiving unit.

9. A calculating apparatus comprising a calculating unit that calculates a value of a formula for evaluating a state of inflammation in a ruminant after parturition, using (i) at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, and (ii) the formula including an explanatory variable to be substituted with the at least one value.

10. An evaluating program including programmed instructions for causing an information processing apparatus to execute an evaluating method comprising an evaluating step of evaluating a state of inflammation in a ruminant after parturition, using at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, or using a value of a formula calculated using the at least one value and the formula including an explanatory variable to be substituted with the at least one value.

11. A calculating program including programmed instructions for causing an information processing apparatus to execute a calculating method comprising a calculating step of calculating a value of a formula for evaluating a state of inflammation in a ruminant after parturition, using (i) at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, and (ii) the formula including an explanatory variable to be substituted with the at least one value.

12. A computer-readable recording medium that stores the program according to claim 10 or 11.

13. An evaluating system comprising an evaluating apparatus and a terminal apparatus that are connected to each other communicatively via a network, wherein
the terminal apparatus includes:
a data sending unit that transmits, to the evaluating apparatus, (i) at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of a ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, or (ii) a value of a formula calculated using the at least one value and the formula including an explanatory variable to be substituted with the at least one value; and
a result receiving unit that receives an evaluation result on a state of inflammation after parturition transmitted from the evaluating apparatus, and
the evaluating apparatus includes:
a data receiving unit that receives the at least one value or the value of the formula transmitted from the terminal apparatus;
an evaluating unit that evaluates the state of the inflammation in the ruminant after parturition, using the at least one value or the value of the formula received by the blood data receiving unit; and
a result sending unit that transmits the evaluation result obtained by the evaluating unit to the terminal apparatus.

14. A terminal apparatus comprising a result obtaining unit that obtains an evaluation result on a state of inflammation after parturition, wherein
the evaluation result is a result of evaluating the state of the inflammation in a ruminant after parturition, using at least one value of concentration values of Ala, Arg, Asn, Asp, BCAA, Cit, Cys, Glu, Gln, Gly, His, Ile, Leu, Lys, Met, 3MeHis, Orn, Phe, Pro, Ser, Tau, Thr, Trp, Tyr, and Val in blood of the ruminant before parturition, concentration values of ALB, ALT, AST, BHBA, BUN, Ca, gGTP, Glc, Glob, NEFA, T-Bil, TCHO, TG, TP, P, Na, K, Cl, GGT, ALP, LDH, CPK, LDL-C, HDL-C, Mg, AMY, PA, LA, CRE, LBP, IL-1β, haptoglobin, serum amyloid A, IL-6, TNF-α, cortisol, and kynurenine in the blood, and a d-ROMs value, a BAP value, a BAP value/d-ROMs value, and a d-ROMs value/BAP value obtained from the blood, or using a value of a formula calculated using the at least one value and the formula including an explanatory variable to be substituted with the at least one value.

15. The terminal apparatus according to claim 14, wherein
the terminal apparatus is communicatively connected via a network to an evaluating apparatus that evaluates a state of inflammation after parturition,
the terminal apparatus further comprises a data sending unit that transmits the at least one value or the value of the formula to the evaluating apparatus, and
the result obtaining unit receives the evaluation result transmitted from the evaluating apparatus.

16. The evaluating method according to claim 1, further comprising a proposing step of proposing a prophylactic measure for a cow evaluated at the evaluating step as having a high possibility of suffering from the metabolic disease after parturition.

17. The evaluating method according to claim 16, wherein the prophylactic measure is at least one selected from the group consisting of administration of Rumen Protect amino acid, administration of a feed additive, administration of a drug, and veterinary diagnosis.

18. The evaluating method according to claim 17, wherein the feed additive is at least one selected from the group consisting of PH adjusters, ion balance adjusters, mycotoxin adsorbents, propionate analogs such as calcium propionate, vitamins, minerals, amino acids, fatty acids, urea, probiotics, yeasts, enzymes, antibiotics, antioxidants, antibacterial agents, and organic acids.

19. The evaluating method according to any one of claims 16 to 18, wherein at the proposing step, statistical causal inference is performed to infer a cause of disease for a cow evaluated as having a possibility of suffering from the metabolic disease, and a prophylactic measure corresponding to the cause is proposed.
